# EUROPEAN PATENT APPLICATION

(11) **EP 4 640 670 A1**
(43) Date of publication of application: **29.10.2025**
(21) Application number: 23907151.7
(22) Date of filing: 21.12.2023
(51) Int. Cl.: C07C 279/04, C01B 25/44, C07C 279/08, C07C 279/12, C07C 323/44, C07F 7/18, C07F 9/117, C08B 37/02, C08B 37/04, C08B 37/08, C08B 37/10, C08B 37/16

(54) **MECHANICALLY-STURDY SUSTAINABLE PLASTIC, AND GREEN AND NONCOVALENT PRODUCTION METHOD OF SAME**

(30) Priority: 21.12.2022 JP 2022204315
(71) Applicant: The University of Tokyo, Bunkyo-ku, Tokyo 113-8654 (JP)
(72) Inventor: AIDA, Takuzo, Tokyo 113-8654 (JP); HUANG, Hubiao, Tokyo 113-8654 (JP); CHENG, Yiren, Tokyo 113-8654 (JP); HIRANO, Eiji, Tokyo 113-8654 (JP)
(74) Representative: Maiwald GmbH
(86) International application number: PCT/JP2023/046016
(87) International publication number: WO 2024/135791

(57) **Abstract**

Provided is a composite including: an organic cation, which is obtained by ionization of a compound having at least two amino groups or guanidino groups; and an oxyanion, wherein the organic cation and the oxyanion are bonded to each other through an ionic bond and a hydrogen bond.

## Description

### Technical Field

The present invention relates to a mechanically-robust sustainable plastic and a method of producing the plastic.

### Background Art

In our daily lives, various plastics, such as polyethylene terephthalate, polycarbonate, polymethacrylate, polyolefin, and polyurethane, have been used. The amount of the plastics produced from 1950 onward exceeds 8.3 billion tons, and 6.3 billion tons out of the plastics have been disposed of as wastes (Non-patent Literature 1). The influences of used plastics on environments, such as an ocean and soil, have become problems.

Both within and outside Japan, laws on the regulation of plastics, such as the Basel Law in 2021 on the import and export of plastics, and the "Act on Promotion of Resource Circulation for Plastics" in Japan, have been executed to advance the regulation of the plastics. The utilization of a low-environmental load plastic has been required.

Although the recycling of the plastics has been promoted, even in the world, the recycling rate thereof is merely about 9% of the production amount thereof (Non-patent Literature 1), and a large part of the used plastics have still been incinerated. Although the recycling of polyethylene terephthalate has been relatively advancing, it is difficult to degrade and recycle the polyethylene terephthalate.

Various kinds of polymers (Non-patent Literature 2) that can each be recycled with a catalyst and biodegradable polymers (Non-patent Literature 3) have been developed. However, the recycling of the plastics involves such problems as described below: the recycling requires many steps, and hence leads to high costs; a catalyst such as a precious metal is used in the recycling of the plastics, and hence leads to high costs; and it is difficult to degrade the plastics into monomers.

Meanwhile, in a supramolecular polymer in which monomers are bonded to each other through a noncovalent bond, the monomers adhere to each other through a weak reversible interaction. The polymer can be recycled because of, for example, the following reason: even when the monomers are separated from each other or a bond therebetween is broken by an external stimulus, the monomers adhere to each other again through self-repair. Accordingly, the polymer has been expected as a new low-environmental load material. In, for example, Non-patent Literature 4, there is a disclosure of a photoresponsive supramolecular polymer glass formed by the aggregation of the monomers of 1,1,1-tris(hydroxymethyl)propane having three ureido-4-pyrimidinone groups, the glass having high stiffness and satisfactory self-repairability. In Non-patent Literature 5, there is a disclosure of a metal supramolecular copolymer formed by combining a monomer, which is obtained by bonding three 2,6-bis(1'-methylbenzimidazolyl)pyridine molecules to a 1,3,5-tris(alkyl)benzene core, or a monomer, which is obtained by bonding two 2,6-bis(1'-methylbenzimidazolyl)pyridine molecules to poly(ethylene-co-butylene), with Zn(NTf₂)₂. However, the related-art photoresponsive supramolecular polymer glass is poor in mechanical strength.

### Citation List

### Non-patent Literature

NPL 1: Sci. Adv. 2017, 3, e1700782.
NPL 2: Science 2018, 360, 398-403.
NPL 3: Nature 2020, 590, 423-427.
NPL 4: Nature Commun. 2016, 7, 10995.
NPL 5: Nature Commun. 2022, 13, 356.

### Summary of Invention

### Technical Problem

A problem to be solved by the present invention is to provide a dynamically-robust composite that can be greenly synthesized and molded with a water solvent.

### Solution to Problem

The present invention encompasses the following embodiments.

### Item 1.

A composite including:
an organic cation, which is obtained by ionization of a compound having at least two amino groups or guanidino groups; and
an oxyanion,
wherein the organic cation and the oxyanion are bonded to each other through an ionic bond and a hydrogen bond.

### Item 2.

The composite according to Item 1, wherein the organic cation and the oxyanion are bonded to each other through an ionic bond and a hydrogen bond represented by one or two or more kinds out of the following formulae (1) to (4): 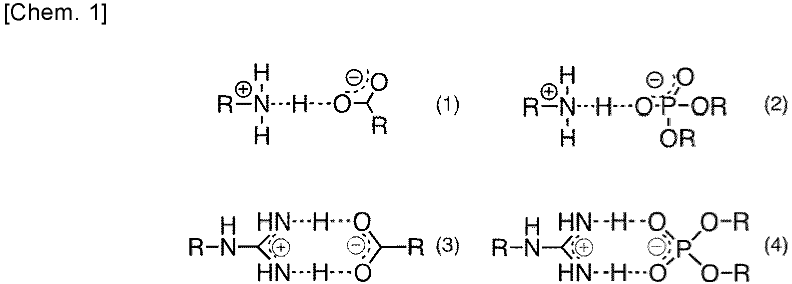 in each of the formulae (1) to (4), R represents an arbitrary monovalent organic group.

### Item 3.

The composite according to Item 1, wherein the organic cation is an organic cation obtained by ionization of a guanidine compound represented by the following formula (6): 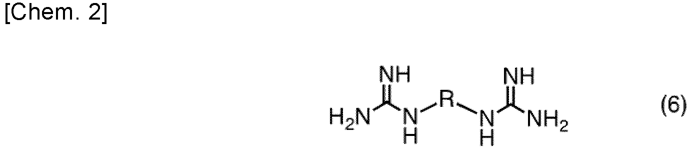 where R represents a substituted or unsubstituted hydrocarbon chain, and
when the hydrocarbon chain is substituted, part of methylene groups of the hydrocarbon chain are each substituted with a group selected from the group consisting of: -NH-; a -N(alkyl group)-; -O-; -COO-; -O-COO-; -NHCO-; -S-; a cycloalkane; a cycloalkanone; benzene; a group represented by the formula (7); and a substituted or unsubstituted -N(guanidylalkylene group)-, and when the - N(guanidylalkylene group)- is substituted, part of methylene groups of a guanidylalkylene group are each substituted with the same group as the group substituting each of part of the methylene groups of the hydrocarbon chain; 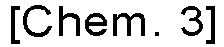 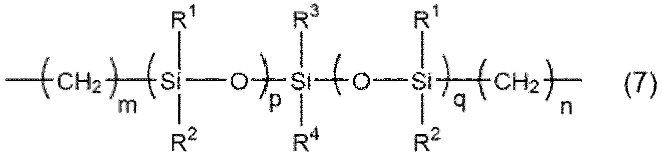 where R₁ and R₂ each independently represent an alkyl group having 1 to 6 carbon atoms or a phenyl group, R₃ and R₄ each independently represent an alkyl group having 1 to 6 carbon atoms or a phenyl group, "m" represents from 1 to 6, "n" represents from 1 to 6, "p" represents an integer in a range of from 0 to 20, and "q" represents an integer in a range of from 0 to 20, provided that p+q is an integer of 1 or more.

### Item 4.

The composite according to Item 3, wherein the guanidine compound contains the following compound (I), the following compound (II), or both thereof:
(I) a compound represented by the formula (6) in which A represents a substituted or unsubstituted hydrocarbon chain, and when the hydrocarbon chain is substituted, part of methylene groups of the hydrocarbon chain are each substituted with a group selected from the group consisting of: -NH-; a -N(alkyl group)-; -O-; -COO-; -O-COO-; - NHCO-; -S-; a cycloalkane; a cycloalkanone; benzene; and a substituted or unsubstituted -N(guanidylalkylene group)-, and when the -N(guanidylalkylene group)- is substituted, part of methylene groups of a guanidylalkylene group are each substituted with the same group as the group substituting each of part of the methylene groups of the hydrocarbon chain, provided that a compound in which part of the methylene groups of the hydrocarbon chain are each substituted with a group represented by the formula (7) is excluded; and
(II) a compound represented by the formula (6) in which A represents a substituted hydrocarbon chain, and part of methylene groups of the hydrocarbon chain are each substituted with a group represented by the formula (7).

### Item 5.

The composite according to Item 1, wherein the oxyanion is an oxyanion of sulfur, phosphorus, silicon, or carbon.

### Item 6.

The composite according to Item 5, wherein the oxyanion is a polyoxyanion.

### Item 7.

The composite according to Item 5, wherein the oxyanion is a cyclic phosphate anion represented by the following formula (10), a linear phosphate anion represented by the following formula (11), an anion of phytic acid, or an anion of a carboxylic acid: 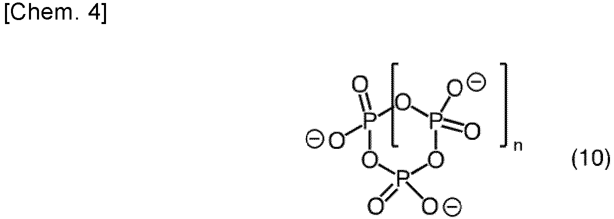 where "n" represents 1 or 4; 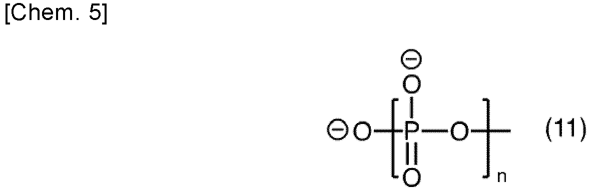 where "n" represents an integer of from 1 to 1,000.

### Item 8.

The composite according to Item 1 or 5, wherein the oxyanion is an oxyanion produced by electrolytic dissociation of a polysaccharide having an anionic functional group.

### Item 9.

The composite according to Item 1, wherein the composite is insoluble in an organic solvent.

### Item 10.

The composite according to Item 1, wherein the composite is processable in water at 20°C.

### Item 11.

The composite according to Item 1, wherein the composite has self-repairability.

### Item 12.

The composite according to Item 1, wherein the composite having a thickness of 0.5 mm has a light transmittance of 90% or more at from 400 nm to 800 nm.

### Item 13.

The composite according to Item 1, wherein the composite is a supramolecular plastic.

### Item 14.

The composite according to Item 1, wherein the composite is a supramolecular polymer glass.

### Item 15.

A composition including the composite of any one of Items 1 to 14.

### Item 16.

An article including the composite of any one of Items 1 to 14.

### Item 17.

A method of producing a composite, the method including mixing a compound having at least two amino groups or guanidino groups and an oxyanion-containing compound in water or an aqueous solution to produce a composite in which an organic cation formed by ionization of the compound having at least two amino groups or guanidino groups, and an oxyanion formed by ionization of the oxyanion-containing compound are bonded to each other through an ionic bond and a hydrogen bond.

### Item 18.

A use of a compound having at least two amino groups or guanidino groups and an oxyanion-containing compound of sulfur, phosphorus, silicon, or carbon for production of a supramolecular polymer composite.

### Advantageous Effects of Invention

According to the present invention, there can be provided the dynamically-robust composite that has a small or no environmental load in its production and processing.

### Brief Description of Drawings

FIG. 1 is a schematic view for illustrating the polymer network of a supramolecular polymer glass (SPG).
FIG. 2A is a photograph showing a liquid-liquid phase separation at the time of the production of the supramolecular polymer glass (SPG).
FIG. 2B is a microphotograph of the micelles of a supramolecular polymer.
FIG. 3 is a graph showing the light transmittance of each of various plastics and a first-generation supramolecular polymer glass. The term "PMMA" means polymethyl methacrylate, the term "PC" means polycarbonate, the term "PET" means polyethylene terephthalate, the term "PS" means polystyrene, the term "Glass" means an inorganic glass, and the term "SPG" means the supramolecular polymer glass.
FIG. 4 is a photograph showing a state in which a weight is mounted on the first-generation supramolecular polymer glass molded by hot pressing.

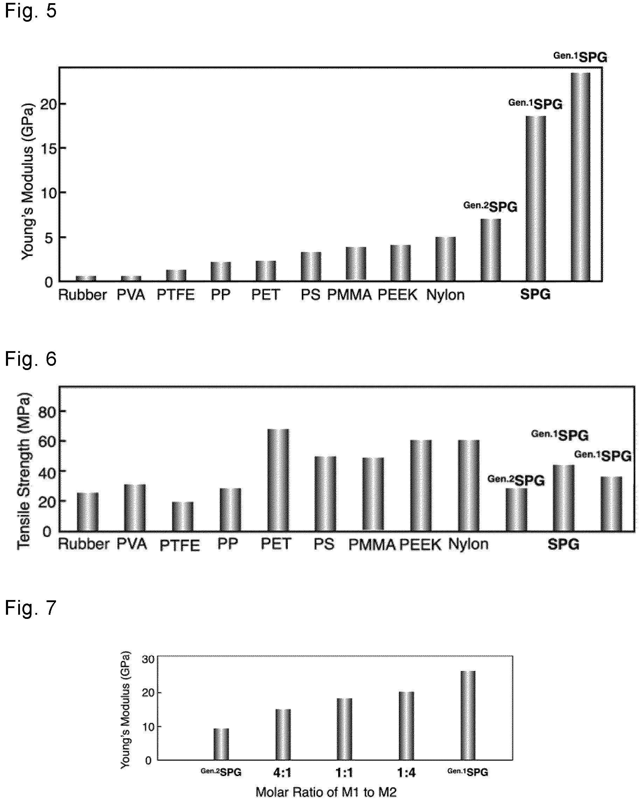

FIG. 5 is a graph showing the Young's moduli of various plastics and first-generation supramolecular polymer glasses. In the second bar from the right, a first-generation monomer ^{Gu}M^{Gen.I}-(9-2) is used. In the rightmost bar, a first-generation monomer ^{Gu}M^{Gen.I}-(9-4) is used. The term "Rubber" means a rubber, the term "PVA" means polyvinyl acetate, the term "PTFE" means polytetrafluoroethylene, the term "PP" means polypropylene, the term "PET" means the polyethylene terephthalate, the term "PS" means the polystyrene, the term "PMMA" means the polymethyl methacrylate, the term "PEEK" means an aromatic polyetherketone, the term "Nylon" means nylon, and the term "SPG" means a supramolecular polymer glass.
FIG. 6 is a graph showing the tensile strengths of the various plastics and the first-generation supramolecular polymer glasses. In the second bar from the right, the first-generation monomer ^{Gu}M^{Gen.I}-(9-2) is used. In the rightmost bar, the first-generation monomer ^{Gu}M^{Gen.I}-(9-4) is used. The term "Rubber" means the rubber, the term "PVA" means the polyvinyl acetate, the term "PTFE" means the polytetrafluoroethylene, the term "PP" means the polypropylene, the term "PET" means the polyethylene terephthalate, the term "PS" means the polystyrene, the term "PMMA" means the polymethyl methacrylate, the term "PEEK" means the aromatic polyetherketone, the term "Nylon" means the nylon, and the term "SPG" means a supramolecular polymer glass.
FIG. 7 is a graph of the Young's moduli of the first-generation supramolecular polymer glass (^{Gen.1}SPG, the rightmost bar), a second-generation supramolecular polymer glass (^{Gen.2}SPG, the leftmost bar), and three third-generation supramolecular polymer glasses (^{Gen.2}SPG and ^{Gen.1}_{SPG}, three central bars). A mixing molar ratio between guanidine compounds (referred to as "M1" and "M2") used in the production of each of the third-generation supramolecular polymer glasses is changed (molar ratios "M1:M2" of 4:1, 1:1, and 1:4 are arranged from the left to the right).
FIG. 8A and FIG. 8B are each a photograph showing the underwater processability of the first-generation supramolecular polymer glass. FIG. 8A is a photograph showing a state 10 seconds after the loading of the glass into water, and FIG. 8B is a photograph showing a state 2 hours after the loading into the water.
FIG. 9 is a set of a view and a photograph of the synthesis of a supramolecular polymer glass through use of a diamine and sodium hexametaphosphate.
FIG. 10A is a graph showing the measurement results of the indentation experiment of a SPG formed of ^{Gu}M^{Gen.II}-1 and phytic acid. The term "Force" means a force and the term "Displacement" means a displacement.
FIG. 10B is a graph showing the measurement results of the indentation experiment of a SPG formed of ^{Gu}M^{Gen.I}-2 and phytic acid. The term "Force" means a force and the term "Displacement" means a displacement.
FIG. 11A is a graph showing the measurement results of the indentation experiment of a SPG formed of a diamine and alginic acid.
FIG. 11B is a graph showing the measurement results of the indentation experiment of a SPG formed of guanidine and alginic acid.
FIG. 11C is a graph showing the measurement results of the indentation experiment of a SPG formed of a diamine/guanidine, alginic acid, and hexametaphosphoric acid.
FIG. 12 is a photograph of a supramolecular polymer emulsion produced by mixing chondroitin sulfate and a first-generation monomer (^{Gu}M^{Gen.I}).
FIG. 13 is a photograph of a supramolecular polymer glass synthesized in Example 6.
FIG. 14 is a photograph of a supramolecular polymer emulsion produced by mixing sodium heparin sulfate and the first-generation monomer (^{Gu}M^{Gen.I}).
FIG. 15 is a photograph of a supramolecular polymer glass synthesized in Example 7.
FIG. 16 is a photograph of a supramolecular polymer emulsion produced by mixing sodium dextran sulfate and the first-generation monomer (^{Gu}M^{Gen.I}).
FIG. 17 is a photograph of a supramolecular polymer glass synthesized in Example 8.
FIG. 18 is a photograph of a supramolecular polymer emulsion produced by mixing β-cyclodextrin substituted with -SO₃Na and the first-generation monomer (^{Gu}M^{Gen.I}). An inserted photograph on the left side shows a glass tube containing the emulsion.
FIG. 19 is a photograph of a supramolecular polymer glass synthesized in Example 9.

### Description of Embodiments

As used herein, the terms "contain" and "include" are each a concept encompassing "consist of."

In numerical ranges described herein in stages, the upper limit value or lower limit value of a numerical range at a certain stage may be freely combined with the upper limit value or lower limit value of a numerical range at any other stage. In addition, in a numerical range described herein, the upper limit value or lower limit value of the numerical range may be replaced with a value described in Examples or a value that may be uniquely derived from Examples. Further, in this description, numerical values tied to each other through the term "to" mean a numerical range including the numerical values in front of and behind the term "to" as a lower limit value and an upper limit value.

As used herein, the term "composite" refers to a material formed by bonding two or more kinds of substances. The respective substances for forming the composite are sometimes referred to as "monomers". A composite formed by bonding two or more kinds of molecules is sometimes referred to as "molecular assembly."

As used herein, the term "supramolecular polymer" refers to a polymer obtained by bonding two or more kinds of monomers through a reversible interaction. The reversible interaction is, for example, a noncovalent bond, such as a hydrogen bond, an ionic bond, a hydrophobic interaction, an electrostatic interaction, and/or a van der Waals force.

As used herein, the term "supramolecular plastic" refers to a polymer obtained by bonding two or more kinds of monomers through a reversible interaction or a composition containing the polymer. The "supramolecular plastic" may be the same as the "supramolecular polymer," or may contain a substance except the "supramolecular polymer." The "supramolecular plastic" may be a synthetic resin.

As used herein, the term "glass" refers to a non-crystalline solid material. The term "non-crystalline" is also referred to as "amorphous", and means that no clear diffraction phenomenon is observed by an X-ray diffraction method, and hence the material has a disordered atomic arrangement.

As used herein, the term "organic cation" refers to a cation formed of a structure containing at least one carbon atom.

As used herein, the term "oxyanion" refers to an anion having oxygen bonded to a nonmetal.

According to a first aspect of the present disclosure, there is provided a composite including: an organic cation, which is obtained by the ionization of a compound having at least two amino groups or guanidino groups; and an oxyanion. The organic cation and the oxyanion are bonded to each other through an ionic bond and a hydrogen bond.

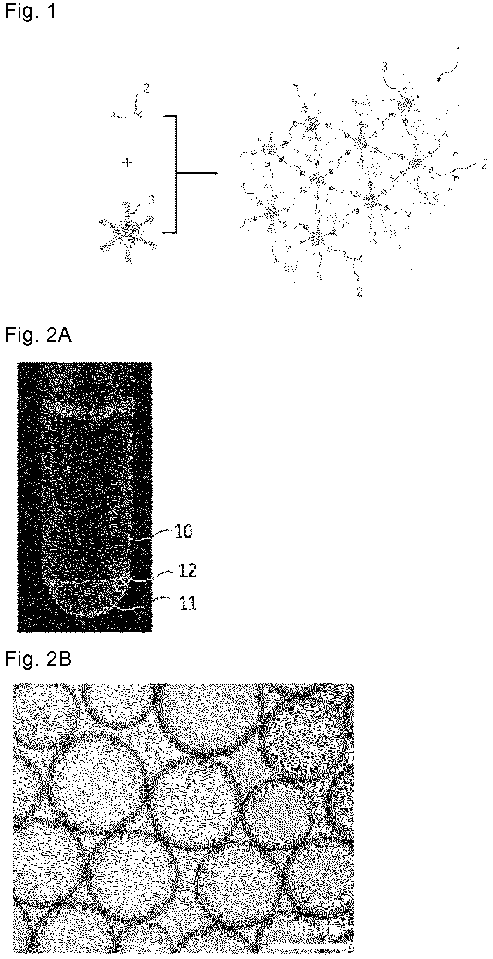

To facilitate the understanding of the invention, an example of a composite that is a supramolecular polymer is schematically illustrated in FIG. 1. The physical properties of the supramolecular polymer to be described below may be applied to the composite of the present invention.

In a supramolecular polymer (1), an organic cation (2), which is obtained by the ionization of a compound having at least two amino groups or a compound having at least two guanidino groups, and an oxyanion (3) are bonded to each other through a noncovalent bond. Accordingly, the bond between those monomers is strong, and hence the mechanical strength of the supramolecular polymer is higher than that of a related-art supramolecular polymer. In addition, the polymer is excellent in recyclability because the organic cation and the oxyanion are easily separated from each other as compared to a case in which the monomers are covalently bonded to each other. The organic cation and the oxyanion may be separated from each other by, for example, being immersed in water or an aqueous solution that is a polar medium for a certain time period or more. The compound having at least two amino groups excludes the compound having at least two guanidino groups.

In some embodiments, the organic cation and the oxyanion are bonded to each other through an ionic bond and a hydrogen bond represented by one or two or more kinds out of the following formulae (1) to (4).

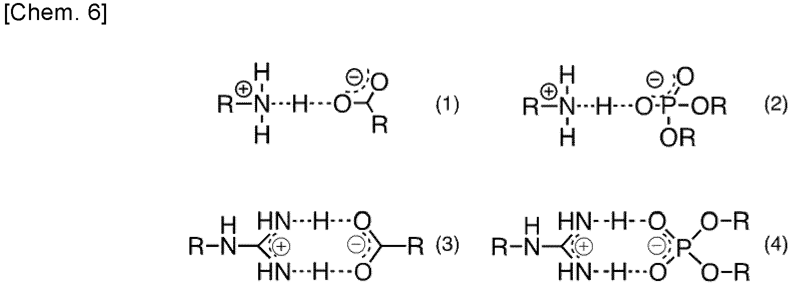

In each of the formulae (1) to (4), R represents an arbitrary monovalent organic group. The term "organic group" as used herein refers to a group having one or more carbon atoms. When two or more Rs are present in one molecule, the Rs may be identical to or different from each other.

As represented by each of the formulae (1) and (2), a hydrogen bond is formed between hydrogen derived from each of the two amino groups of an amine compound and the oxygen of the oxyanion, and an ammonium cation and the oxyanion form an ionic bond therebetween. Alternatively, as represented by each of the formulae (3) and (4), a hydrogen bond is formed between hydrogen derived from each of the two guanidino groups of a guanidine compound and the oxygen of the oxyanion, and a guanidium cation and the oxyanion form an ionic bond therebetween. Accordingly, the mechanical strength of the supramolecular polymer is improved. Meanwhile, the bond between the organic cation and the oxyanion is easy to separate as compared to a covalent bond.

In some embodiments, the compound having at least two amino groups is an amine compound represented by the following formula (5).

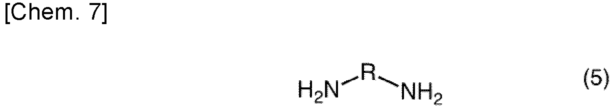

In the formula, R represents a substituted or unsubstituted hydrocarbon chain, and when the hydrocarbon chain is substituted, part of methylene groups of the hydrocarbon chain are each substituted with a group selected from the group consisting of: -NH-; a -N(alkyl group)-; -O-; -COO-; -O-COO-; -NHCO-; -S-; a cycloalkane; a cycloalkanone; benzene; a group represented by the formula (7); and a substituted or unsubstituted -N(guanidylalkylene group)-, and when the -N(guanidylalkylene group)- is substituted, part of methylene groups of a guanidylalkylene group are each substituted with the same group as the group substituting each of part of the methylene groups of the hydrocarbon chain.

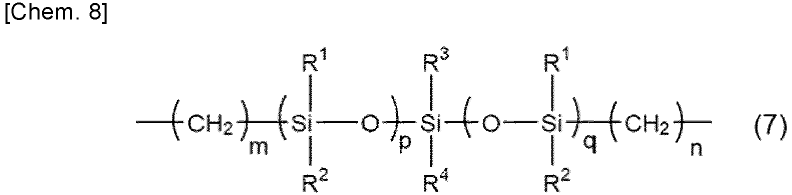

In the formula, R₁ and R₂ each independently represent an alkyl group having 1 to 6 carbon atoms or a phenyl group, R₃ and R₄ each independently represent an alkyl group having 1 to 6 carbon atoms or a phenyl group, "m" represents from 1 to 6, "n" represents from 1 to 6, "p" represents an integer in the range of from 0 to 20, and "q" represents an integer in the range of from 0 to 20, provided that p+q is an integer of 1 or more.

The hydrocarbon chain represented by R may be an aliphatic hydrocarbon chain, an alicyclic hydrocarbon chain, an aromatic hydrocarbon chain, or a combination thereof. The aliphatic hydrocarbon chain may be a saturated and linear aliphatic hydrocarbon chain or an unsaturated aliphatic hydrocarbon chain, and may be linear or branched. The hydrocarbon chain is preferably a linear or branched and saturated hydrocarbon chain.

When part of the methylene groups of the hydrocarbon chain are substituted, the number of the methylene groups to be substituted is not limited. However, the number is preferably from 1 to 20, more preferably from 1 to 10, still more preferably from 1 to 5.

In a specific embodiment, the alkyl group of the -N(alkyl group) that is a substituent for a methylene group of the hydrocarbon chain is preferably a linear or branched alkyl having 1 to 6 carbon atoms. In a specific embodiment, the alkylene group of the -N(guanidylalkylene group)- that is a substituent for a methylene group of the hydrocarbon chain is preferably a linear or branched alkylene having 1 to 6 carbon atoms.

In the formula (7), the alkyl group having 1 to 6 carbon atoms represented by each of R₁ and R₂ may be linear, branched, or cyclic. The alkyl group having 1 to 6 carbon atoms represented by each of R₃ and R₄ may be linear, branched, or cyclic. In a specific embodiment, the alkyl groups each having 1 to 6 carbon atoms represented by R₁, R₂, R₃, and R₄ are each independently a linear alkyl group having 1 to 6 carbon atoms. In a specific embodiment, "p" and "q" each represent an integer in the range of from 1 to 20. In a specific embodiment, one of "p" or "q" represents an integer in the range of from 1 to 20, and the other of "p" or "q" represents 0.

The compound having at least two amino groups may have two, three, or four or more amino groups in a molecule thereof.

In the case where the amine compound has three amino groups in a molecule thereof, the mechanical strength of the supramolecular polymer typically increases as compared to that in the case where the compound has two amino groups in a molecule thereof. In addition, when the guanidine compound has a substituent based on -NH- on nitrogen in a hydrocarbon chain thereof, the polymer is improved in water solubility, and easily has an amorphous shape.

In some embodiments, the compound having at least two guanidino groups is a guanidine compound represented by the following formula (6).

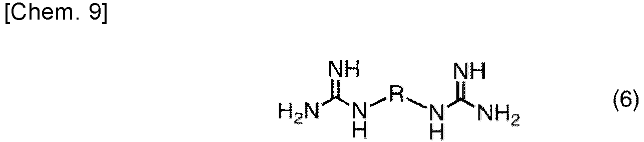

In the formula, R represents a substituted or unsubstituted hydrocarbon chain, and when the hydrocarbon chain is substituted, part of methylene groups of the hydrocarbon chain are each substituted with a group selected from the group consisting of: -NH-; a -N(alkyl group)-; -O-; -COO-; -O-COO-; -NHCO-; -S-; a cycloalkane; a cycloalkanone; benzene; a group represented by the formula (7); and a substituted or unsubstituted -N(guanidylalkylene group)-, and when the -N(guanidylalkylene group)- is substituted, part of methylene groups of a guanidylalkylene group are each substituted with the same group as the group substituting each of part of the methylene groups of the hydrocarbon chain.

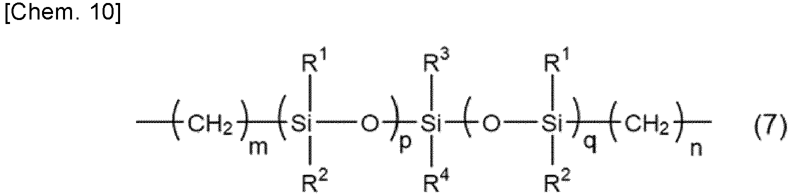

In the formula, R₁ and R₂ each independently represent an alkyl group having 1 to 6 carbon atoms or a phenyl group, R₃ and R₄ each independently represent an alkyl group having 1 to 6 carbon atoms or a phenyl group, "m" represents from 1 to 6, "n" represents from 1 to 6, "p" represents an integer in the range of from 0 to 20, and "q" represents an integer in the range of from 0 to 20, provided that p+q is an integer of 1 or more.

The hydrocarbon chain represented by R may be an aliphatic hydrocarbon chain, an alicyclic hydrocarbon chain, an aromatic hydrocarbon chain, or a combination thereof. The aliphatic hydrocarbon chain may be a saturated and linear aliphatic hydrocarbon chain or an unsaturated aliphatic hydrocarbon chain, and may be linear or branched. The hydrocarbon chain is preferably a linear or branched and saturated hydrocarbon chain.

When part of the methylene groups of the hydrocarbon chain are substituted, the number of the methylene groups to be substituted is not limited. However, the number is preferably from 1 to 20, more preferably from 1 to 10, still more preferably from 1 to 5.

In a specific embodiment, the alkyl group of the -N(alkyl group) that is a substituent for a methylene group of the hydrocarbon chain is preferably a linear or branched alkyl having 1 to 6 carbon atoms. In a specific embodiment, the alkylene group of the -N(guanidylalkylene group)- that is a substituent for a methylene group of the hydrocarbon chain is preferably a linear or branched alkylene having 1 to 6 carbon atoms.

In the formula (7), the alkyl group having 1 to 6 carbon atoms represented by each of R₁ and R₂ may be linear, branched, or cyclic. The alkyl group having 1 to 6 carbon atoms represented by each of R₃ and R₄ may be linear, branched, or cyclic. In a specific embodiment, the alkyl groups each having 1 to 6 carbon atoms represented by R₁, R₂, R₃, and R₄ are each independently a linear alkyl group having 1 to 6 carbon atoms. In a specific embodiment, "p" and "q" each represent an integer in the range of from 1 to 20. In a specific embodiment, one of "p" or "q" represents an integer in the range of from 1 to 20, and the other of "p" or "q" represents 0.

The guanidine compound having at least two guanidino groups may have two, three, or four or more guanidino groups in a molecule thereof.

In the case where the guanidine compound has three guanidino groups in a molecule thereof, the mechanical strength of the supramolecular polymer typically increases as compared to that in the case where the compound has two guanidino groups in a molecule thereof. In addition, when the guanidine compound has a substituent based on -NH- on nitrogen in a hydrocarbon chain thereof, the polymer is improved in water solubility, and easily has an amorphous shape.

In some embodiments, the above-mentioned guanidine compound is the following guanidine compound (I) or the following guanidine compound (II), or includes both the guanidine compound (I) and the guanidine compound (II).

### Guanidine Compound (I):

A compound represented by the formula (6) in which A represents a substituted or unsubstituted hydrocarbon chain, and when the hydrocarbon chain is substituted, part of methylene groups of the hydrocarbon chain are each substituted with a group selected from the group consisting of: -NH-; a -N(alkyl group)-; -O-; -COO-; -O-COO-; -NHCO-; -S-; a cycloalkane; a cycloalkanone; benzene; and a substituted or unsubstituted -N(guanidylalkylene group)-, and when the -N(guanidylalkylene group)- is substituted, part of methylene groups of a guanidylalkylene group are each substituted with the same group as the group substituting each of part of the methylene groups of the hydrocarbon chain, provided that a compound in which part of the methylene groups of the hydrocarbon chain are each substituted with a group represented by the formula (7) is excluded.

### Guanidine Compound (II):

A compound represented by the formula (6) in which A represents a substituted hydrocarbon chain, and part of the methylene groups of the hydrocarbon chain are each substituted with a group represented by the formula (7).

A supramolecular polymer produced by using the guanidine compound (I) has a large mechanical strength, but is easily dissolved in water, and hence has high underwater processability.

Particular specific examples of an organic cation obtained by protonating the guanidine compound (I) include the following organic cations.

Although a supramolecular polymer produced by using the guanidine compound (II) has water solubility lower than that of the supramolecular polymer produced by using the guanidine compound (I), its mechanical strength tends to be small.

Accordingly, a supramolecular polymer produced by using both the guanidine compound (I) and the guanidine compound (II) can improve the mechanical strength of the supramolecular polymer produced by using the guanidine compound (II) while suppressing the water solubility of the supramolecular polymer produced by using the guanidine compound (I).

In the supramolecular polymer produced by using both the guanidine compound (I) and the guanidine compound (II), the combination of any guanidine compound (I) and any guanidine compound (II) that are disclosed herein may be used as the guanidine compound (I) and the guanidine compound (II). The number of the kinds of each of the guanidine compounds (I) and the guanidine compounds (II) may be one or two or more.

Although the molar ratio of the guanidine compound (I) to the guanidine compound (II) for producing a supramolecular polymer is not particularly limited, the ratio is preferably from 90:10 to 10:90, more preferably from 20:80 to 80:20.

Particular specific examples of an organic cation obtained by protonating the guanidine compound (II) include organic cations represented by the following formulae (9-1) to (9-10): 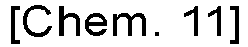 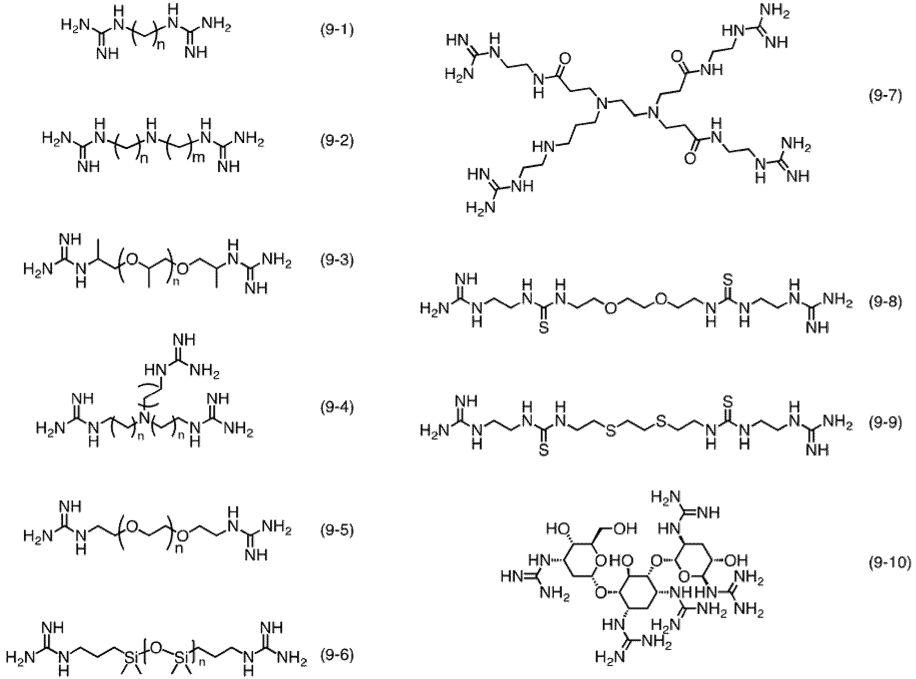 in each of the formulae (9-1) to (9-6), "n" represents an integer of from 1 to 100; and
in the formula (9-2), "m" represents an integer of from 1 to 100.

Guanidine is a high-safety substance present even in a living organism. The guanidine compounds may each be synthesized by a known method, or commercial products may be utilized.

The term "oxyanion" refers to an anion having a structure formed of a central element and oxygen bonded to the central element. The central element is not particularly limited, and may be a metal element or a nonmetal. However, sulfur, phosphorus, silicon, or carbon is more preferred in terms of safety or a low load on an environment.

In some embodiments, the oxyanion is an oxyanion of sulfur, phosphorus, silicon, or carbon. The oxyanion is produced by the electrolytic dissociation of an oxyanion-containing compound of sulfur, phosphorus, silicon, or carbon. The oxyanion-containing compound of sulfur, phosphorus, silicon, or carbon is, for example, but not limited to, a salt selected from the group consisting of: a sulfuric acid salt; a sulfurous acid salt; a sulfonic acid salt; a protonated phosphoric acid salt; a phosphoric acid salt; a polyphosphoric acid salt; a metaphosphoric acid salt; a phosphonous acid salt; an oxyphosphoric acid salt; a silicic acid salt; a carboxylic acid salt; a carbonic acid salt; and combinations thereof. The salt is preferably a metal salt, and preferred examples of a metal for forming the metal salt include, but not limited to, sodium, potassium, lithium, calcium, strontium, barium, and magnesium. One kind of oxyanion may be incorporated into the supramolecular polymer, or two or more kinds of oxyanions may be incorporated thereinto. Alternatively, the oxyanion-containing compound of sulfur, phosphorus, silicon, or carbon is, for example, but not limited to, a salt selected from the group consisting of: a sulfuric acid ester; a phosphodiester; a silicic acid ester; a carboxylic acid ester; and combinations thereof.

In some embodiments, the above-mentioned oxyanion is a polyoxyanion having two or more central elements in a molecule thereof. When the oxyanion is an anion that is divalent or more, the oxyanion can be bonded to the above-mentioned organic cation that is a counterpart molecule at two or more sites, and hence the formation of a network structure by the bonding of the above-mentioned organic cation and the above-mentioned oxyanion becomes possible.

In some embodiments, the oxyanion is a cyclic phosphate anion represented by the following formula (10), a linear phosphate anion represented by the following formula (11), an anion of phytic acid, or an anion of a carboxylic acid.

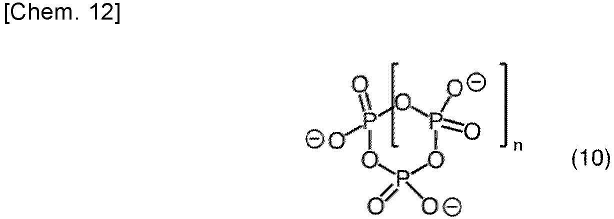

In the formula, "n" represents an integer of from 1 to 4.

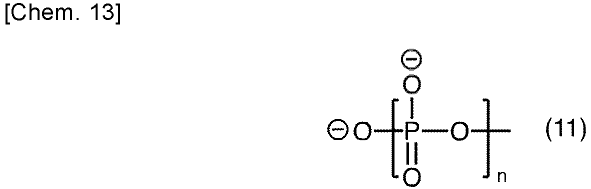

In the formula, "n" represents an integer of from 1 to 1,000.

In the cyclic phosphate anion of the formula (10), "n" preferably represents 1 or 4, and "n" more preferably represents 4. Sodium hexametaphosphate used as a raw material for a hexaphosphate ion when "n" represents 4 is a compound approved by the U.S. Food and Drug Administration (FDA), and hence has high safety. In addition, an oxyanion in which "n" represents 4 is more preferred because a supramolecular polymer having a high mechanical strength is obtained. The upper limit value of "n" is preferably 100.

"n" in the linear phosphate anion represented by the formula (11) preferably represents from 1 to 1,000.

The oxyanion-containing compound of sulfur, phosphorus, silicon, or carbon may be synthesized by a known method, or a commercial product may be utilized.

The anion of phytic acid is an anion produced by the deprotonation of phytic acid represented by the formula (12).

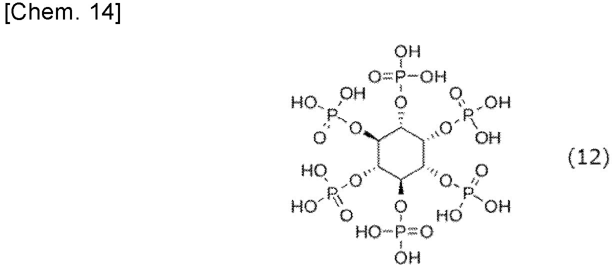

The anion of the carboxylic acid is also referred to as "carbanion", and examples thereof include, but not limited to, anions produced by the deprotonation of carboxylic acids represented by the formulae (13-1) and (13-2), and an anion of alginic acid represented by the formula (14).

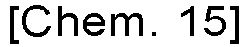

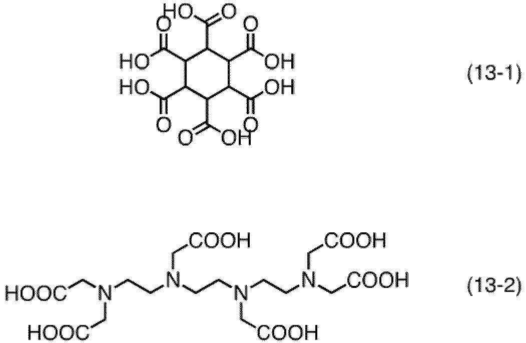

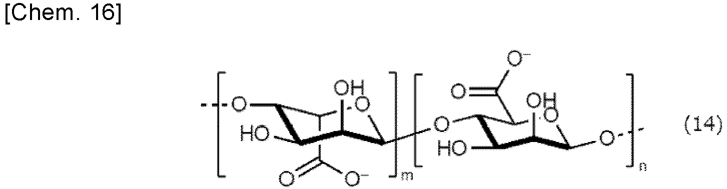

In some embodiments, the oxyanion-containing compound is a polysaccharide, and hence the above-mentioned oxyanion is an oxyanion produced by the electrolytic dissociation of the polysaccharide. The polysaccharide is preferably a polysaccharide having an anionic functional group, more preferably an acidic polysaccharide having an anionic functional group. Examples of such anionic functional group include an acid group, a salt, an acid ester, and combinations thereof. When the anionic functional group is a salt, the salt is, for example, but not limited to, a salt selected from the group consisting of: a sulfuric acid salt; a sulfurous acid salt; a sulfonic acid salt; a protonated phosphoric acid salt; a phosphoric acid salt; a polyphosphoric acid salt; a metaphosphoric acid salt; a phosphonous acid salt; an oxyphosphoric acid salt; a silicic acid salt; a carboxylic acid salt; a carbonic acid salt; and combinations thereof. The salt is preferably a metal salt, and preferred examples of a metal for forming the metal salt include, but not limited to, sodium, potassium, lithium, calcium, strontium, barium, and magnesium. Preferred examples of the anionic functional group include a sulfuric acid group, a sulfuric acid salt, a sulfuric acid ester, a carboxy group, a carboxylic acid salt, a carboxylic acid ester, a sulfo group, a sulfonic acid salt, a sulfonic acid ester, a phosphoric acid group, a phosphoric acid salt, a phosphoric acid ester, a phosphonic acid group, a phosphonic acid salt, a phosphonic acid ester, and combinations thereof. The polysaccharide is more preferably a polysaccharide having at least two anionic functional groups selected from the group consisting of: a sulfuric acid group; a sulfuric acid salt; a sulfuric acid ester salt; a carboxy group; a carboxylic acid salt; a carboxylic acid ester; a sulfo group; a sulfonic acid salt; a sulfonic acid ester; a phosphoric acid group; a phosphoric acid salt; a phosphoric acid ester; a phosphonic acid group; a phosphonic acid salt; a phosphonic acid ester; and a phosphodiester. The kinds of the at least two anionic functional groups may be identical to or different from each other.

The polysaccharide having an anionic functional group preferably contains one anionic functional group per monomer unit serving as a constituent unit for the polysaccharide. All the monomer units of the polysaccharide may each have an anionic functional group, or part of the monomer units may each have an anionic functional group. At least two anionic functional groups may be directly bonded to a carbon-containing five-membered ring or six-membered ring of the monomer unit serving as a constituent unit for the polysaccharide, or may each be bonded to the five-membered ring or the six-membered ring through a substituted or unsubstituted hydrocarbon chain. The hydrocarbon chain is, for example, but not limited to, an alkylene group (e.g., a methylene group).

Groups in the case of the electrolytic dissociation of the at least two functional groups are each, for example, a sulfuric acid ester group (-O-SO₃⁻), a sulfonate group (-SO₃⁻), a carboxylate group (-COO⁻), a phosphate group (-O-PO₃²⁻), or a phosphoryl group (-PO₃²⁻).

The polysaccharide having an anionic functional group may be a natural polysaccharide, or may be a synthetic polysaccharide. The polysaccharide having an anionic functional group may be linear, branched, or cyclic. Examples of the polysaccharide include, but not limited to carboxymethyl cellulose, gellan gum, alginic acid, sulfated alginic acid, carrageenan, xanthan gum, chondroitin sulfate, heparin, hyaluronic acid, pectic acid, gum arabic, agar, tragacanth gum, sodium dextran sulfate, and a sulfated sodium salt of cyclodextrin.

Although the number of the monomer units of the polysaccharide that electrolytically dissociates to produce the oxyanion, in particular, the polysaccharide having an anionic functional group is not particularly limited, the number is preferably from 2 to 100,000.

Although the molecular weight of the polysaccharide that electrolytically dissociates to produce the oxyanion, in particular, the polysaccharide having an anionic functional group is not particularly limited, the weight-average molecular weight thereof is preferably from 1,000 to 10,000,000, more preferably from 5,000 to 1,000,000. The weight-average molecular weight of the polysaccharide may be calculated by gel permeation chromatography (GPC) measurement.

The oxyanion produced from the above-mentioned polysaccharide that electrolytically dissociates to produce the oxyanion, in particular, the polysaccharide having an anionic functional group may be bonded to an organic cation, which is obtained by the ionization of an arbitrary compound having at least two amino groups or guanidino groups described herein, through an ionic bond and a hydrogen bond to form a composite.

In some embodiments, the Young's modulus of the above-mentioned supramolecular polymer measured under the following measurement conditions of an indentation experiment is 5 GPa or more, preferably 10 GPa or more, more preferably 15 GPa or more, still more preferably 20 GPa or more.
Measurement conditions: The supramolecular polymer is cut into a size measuring 1 cm long by 1 cm wide by 0.5 mm thick to produce a sample. The Young's modulus of the sample was determined at a measurement temperature of 20°C with an indentation hardness tester ENT-NEXUS (ELIONIX Inc.). A diamond indenter tip was used in the indentation. A test load was set to 50 mN, a loading time was set to 20,000 msec, a holding time was set to 5,000 msec, and an unloading time was set to 20,000 msec.

Although the Young's moduli of many known synthetic resins polymerized through covalent bonds, such as polytetrafluoroethylene, polypropylene, polyethylene terephthalate, polystyrene, polymethyl methacrylate, and an aromatic polyetherketone, are 5 GPa or less, the Young's modulus of a supramolecular polymer glass (SPG) can be made larger than the Young's moduli of such synthetic resins.

In some embodiments, the tensile strength of the above-mentioned supramolecular polymer measured under the following measurement conditions is preferably 5 MPa or more and 50 MPa or less. Those values are values comparable to the tensile strengths of some known synthetic resins polymerized through covalent bonds, and hence the tensile strength of the supramolecular polymer glass (SPG) can be made equal to or larger than the tensile strengths of such synthetic resins. Measurement conditions: The supramolecular polymer is cut into a size measuring 2 mm long by 35 mm wide by 0.5 mm thick to produce a sample. A test speed was set to 10 mm/s. The sensor of a tensile machine indicated 500 N. A measurement temperature is set to 20°C.

In some embodiments, the supramolecular polymer glass (SPG) can be processed in water at 20°C. In the supramolecular polymer glass (SPG), the organic cation and the oxyanion are bonded to each other through an ionic bond and a hydrogen bond. Accordingly, when the glass is placed in water for a certain time period, the water is bonded to its supramolecular polymer to swell and soften the polymer. Thus, the glass can be processed with a hand or a machine. The addition of an electrolyte such as sodium chloride to water further advances the dissociation of the polymer into monomers. In addition, the supramolecular polymer may be molded into an arbitrary shape, such as a flat plate shape or a spherical shape, by, for example, being dampened with water. In addition, the drying of the molded supramolecular polymer can maintain the shape of the supramolecular polymer after the molding. 100 Percent of the supramolecular polymer of this embodiment may be degradable in water, and such supramolecular polymer is advantageous in that its environmental load is low.

In some embodiments, the supramolecular polymer glass (SPG) can be molded into various shapes at temperatures exceeding its glass transition temperature.

In some embodiments, the supramolecular polymer glass (SPG) has self-repairability. Even in, for example, the case where the supramolecular polymer glass is fractured into two members, when its fractured surfaces are dampened with water, and the two fractured surfaces are brought into contact with each other and are left for a while, the two members are bonded to each other.

In some embodiments, the Young's modulus of the above-mentioned supramolecular polymer at 20°C is 5 GPa or more, preferably 10 GPa or more, more preferably 15 GPa or more, still more preferably 20 GPa or more, and the tensile strength of the above-mentioned supramolecular polymer at 20°C is 5 MPa or more and 50 GPa or less. The supramolecular polymer having such configuration has high rigidity and has a large mechanical strength against its tension.

In some embodiments, the supramolecular polymer glass (SPG) is insoluble in an organic solvent. Examples of the organic solvent include dichloromethane, chloroform, methanol, ethanol, acetone, hexane, dimethylformamide, dimethyl sulfoxide, ethyl acetate, diethyl ether, and tetrahydrofuran.

In some embodiments, the light transmittance of the supramolecular polymer glass having a thickness of 0.5 mm at from 400 nm to 800 nm is 95% or more. Such supramolecular polymer is excellent in transparency.

The supramolecular polymer that is the composite of the first aspect of the present invention has one or a plurality of advantages out of the following advantages [1] to [8]. In a particularly preferred embodiment, the polymer has all the advantages except the advantage [5], or has all the advantages [1] to [8].

### [1] Quantitative Green Synthesis

An organic cation, which is obtained by the ionization of a compound having at least two amino groups or guanidino groups, and an oxyanion are noncovalently bonded to each other at a molar ratio of 1:1 to produce a supramolecular polymer. The supramolecular polymer can be synthesized without need for heating or pressurization. In addition, an organic solvent is not required because the supramolecular polymer can be synthesized in water or an aqueous solvent.

### [2] Green Molding

The supramolecular polymer can be processed in water. Heating for the processing is not required.

### [3] Super Robust

The Young's modulus of the supramolecular polymer at 20°C is 5 GPa or more, and/or the tensile strength thereof at 20°C is 5 MPa or more and 50 GPa or less.

### [4]Self-repair

In the case where the supramolecular polymer is fractured into two members, when its fractured surfaces are dampened with water, and the two fractured surfaces are brought into contact with each other and are left, the two members are bonded to each other.

### [5] Water Resistance

The supramolecular polymer produced by using the guanidine compound (II) has water solubility lower than that of the supramolecular polymer produced by using the guanidine compound (I).

### [6]Organic Solvent Resistance

The supramolecular polymer is insoluble in an organic solvent. Examples of the organic solvent include dichloromethane, chloroform, methanol, ethanol, acetone, hexane, dimethylformamide, dimethyl sulfoxide, ethyl acetate, diethyl ether, and tetrahydrofuran.

### [7] Complete Recycling

Unlike a related-art plastic polymer, the recycling of the SPG of this embodiment does not require any procedure including consuming a catalyst or energy. The immersion of the SPG in an aqueous solution of ammonium chloride or an acid breaks the interaction of a salt bridge between a guanidino group and a phosphodiester group to enable complete return of the SPG to its raw material monomers. The monomers may be recovered by a purification method such as an ion exchange resin. The foregoing enables resource circulation, and hence achieves a microplastic-free ocean. The foregoing changes the common sense of plastics.

In some embodiments, the organic cation and the oxyanion in the composite of the first aspect may be bonded to each other through an additional covalent bond or noncovalent bond except an ionic bond and a hydrogen bond. Such additional covalent bond or noncovalent bond may be formed through the introduction of a functional group into the above-mentioned organic cation and/or the above-mentioned oxyanion by a known method.

In some embodiments, the composite of the first aspect is a supramolecular polymer. In some embodiments, the composite of the first aspect is a supramolecular plastic. In some embodiments, the composite of the first aspect is a supramolecular polymer glass. A case in which the composite is a supramolecular polymer is preferred because an environmental load at the time of its production and processing becomes smaller or is absent.

According to a second aspect of the present disclosure, there is provided a composition including the composite of the first aspect described above. The composition may further include a polymer, such as a synthetic resin, an elastomer, or a rubber. In addition, the composition may further include an additive except the polymer. Examples of the additive include, but not limited to, a synthetic resin, an elastomer, a rubber, a surfactant, a lubricant, a dispersant, an antioxidant, a light stabilizer, a UV absorber, a colorant, an antiseptic, and a flavor.

According to a third aspect of the present disclosure, there is provided an article including the composite of the first aspect described above. The article may include a member except the composite of the first aspect. Examples of the article include, but not limited to, a container, a package, metal machine industrial products (an industrial machine, an electric machine, a precision machine, and an electric machine), a home appliance, information devices, such as a personal computer and a cellular phone, kitchenware, a cleaning utensil, stationery, a toy, sports goods, furniture, clothes, a detergent, a medicine, a cosmetic, a coating, a building material, and vehicles (a light vehicle and a vehicle) and parts thereof.

According to a fourth aspect of the present disclosure, there is provided a method of producing a composite, the method including mixing a compound having at least two amino groups or guanidino groups and an oxyanion-containing compound in water or an aqueous solution to produce a composite in which an organic cation formed by the ionization of the compound having at least two amino groups or guanidino groups, and an oxyanion formed by the ionization of the oxyanion-containing compound are bonded to each other through an ionic bond and a hydrogen bond. The composite may be the composite of the first aspect described above. In some embodiments, the composite is a supramolecular polymer. In some embodiments, the composite is a supramolecular polymer glass.

The compound having at least two amino groups or guanidino groups, the oxyanion-containing compound, and the composite are as described for the composite of the first aspect. In particular, the supramolecular polymer that is the composite of the first aspect may be produced in one stage.

When the compound having at least two amino groups or guanidino groups and the oxyanion-containing compound are mixed in water or an aqueous solution, the organic cation formed by the ionization of the compound having at least two amino groups or guanidino groups, and the oxyanion formed by the ionization of the oxyanion-containing compound are ionically bonded and hydrogen-bonded to each other to form the supramolecular polymer. In addition, an anion produced by the electrolytic dissociation of the compound having at least two amino groups or guanidino groups, and an organic cation produced by the electrolytic dissociation of the oxyanion-containing compound neutralize each other to be dissolved in the water. The supramolecular polymer can be easily separated or recovered from the water or the aqueous solution by known methods, such as centrifugation and recovery, because the supramolecular polymer undergoes a liquid-liquid phase separation with a solvent. The drying of the resultant supramolecular polymer provides a supramolecular polymer that has a high mechanical strength despite the fact that its monomer molecules are noncovalently bonded to each other. The supramolecular polymer may be molded into an arbitrary shape, such as a flat plate shape or a spherical shape, after having been separated or recovered from the water or the aqueous solution. An arbitrary molding method, such as press molding, injection molding, or extrusion molding, may be used as a molding method.

The method of producing a composite of this aspect may be performed in an aqueous system, and is environmentally friendly because the use of an organic solvent can be avoided. In addition, neither heating nor pressurization is required, and hence the composite can be produced at a temperature of from 5°C to 40°C and under an ambient atmosphere or under atmospheric pressure. In addition, the method is cost-saving because the use of an expensive rare earth metal (rare metal) catalyst can be avoided.

According to a fifth aspect of the present disclosure, there is provided a use of a compound having at least two amino groups or guanidino groups and an oxyanion-containing compound of sulfur, phosphorus, silicon, or carbon for the production of a supramolecular polymer composite. The compound having at least two amino groups or guanidino groups, and the oxyanion-containing compound of sulfur, phosphorus, silicon, or carbon are as described for the composite of the first aspect.

The disclosure of all the patent applications and literatures cited herein is incorporated herein in its entirety by reference.

The present invention is more specifically described below by way of Examples. However, the present invention is not limited thereto.

### Examples

### Example 1 Synthesis of Guanidinium-based Monomer (^{Gu}M)

### 1. Synthesis of First-generation Monomer 1 (^{Gu}M^{Gen.I}-1)

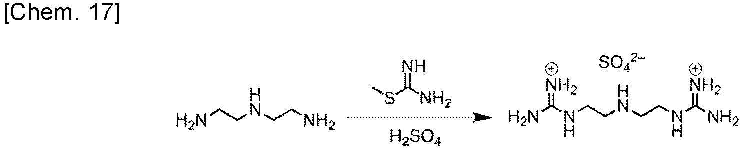

Diethyltriamine (54 mL, 0.5 mol) and S-methylisothiourea·0.5 H₂SO₄ (139.2 g, 1 mol) were added to a mixed solution of water and ethanol (v/v 1:1, 500 mL). The reaction mixture was stirred at room temperature for 16 hours to produce a white precipitate. The crude product was filtered and rinsed with ethanol, followed by recrystallization in a mixture of water and isopropanol. Thus, ^{Gu}M^{Gen.I}-1 was obtained (yield: 93%, based on diethyltriamine). The product was identified by ¹H NMR and ¹³C NMR. ¹H NMR (600 MHz, 298 K, D₂O): δ 3.32 (t, 4H, NH-CH₂-CH₂), 2.81 (t, 4H, CH₂-NH-CH₂) ppm.
¹³C NMR (150 MHz, 298 K, D₂O): δ 157.07 (C=NH), 46.62 (CH₂-NH-CH₂), 40.63 (NH-CH₂-CH₂) ppm.

### 2. Synthesis of First-generation Monomer 2 (^{Gu}M^{Gen.I}-2)

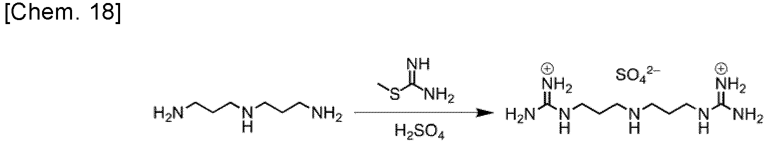

In a 1-liter three-necked round bottom flask that had been dried in an oven, S-methylisothiourea·1/2 H₂SO₄ (250.5 g, 1.8 mol) was dissolved in 700 mL of Milli-Q water. 70 Milliliters of norspermidine was added to the solution. The reaction mixture was refluxed for 72 hours. The reaction mixture was cooled to room temperature. The flask was placed in a freezer at 4°C for 12 hours to produce a white precipitate. The crude product was filtered and washed with ice water, followed by recrystallization with mL of Milli-Q water. Thus, ^{Gu}M^{Gen.I}-2 was obtained (yield: 95%, based on norspermidine). The product was identified by ¹H NMR and ¹³C NMR.
¹H NMR (600 MHz, 298 K, D₂O): δ 2.04 (p, 4H; CH₂CH₂CH₂), 3.16 (t, 4H; CH₂-NH-CH₂), 3.64 (t, 4H; NH-CH₂-CH₂) ppm.
¹³C NMR (150 MHz, 298 K, D₂O): δ 159.73 (C=NH), 47.85 (CH₂-NH-CH₂), 41.04 (NH-CH₂-CH₂), 27.83 (CH₂-CH₂-CH₂) ppm.

### 3. Synthesis of First-generation Monomer 3 (^{Gu}M^{Gen.I}-3)

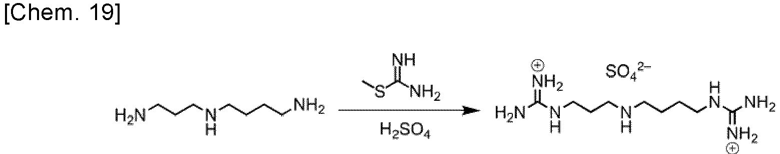

In a 100-milliliter three-necked round bottom flask that had been dried in an oven, S-methylisothiourea·0.5 H₂SO₄ (12.53 g, 0.09 mol) was dissolved in 35 mL of Milli-Q water. 4 Milliliters of spermidine was added to the solution. The reaction mixture was refluxed for 72 hours. The reaction mixture was cooled to room temperature. The crude residue was filtered and washed with ice water, followed by recrystallization with a mixed solution of water and ethanol (v/v 1:1). Thus, ^{Gu}M^{Gen.I}-3 was obtained (yield: 53%, based on spermidine). The product was identified by ¹H NMR and ¹³C NMR.
¹H NMR (600 MHz, 298 K, D₂O): δ 3.31 (t, 2H, NH-CH₂-(CH₂)₂-NH-CH₂), 3.24 (t, 2H, NH-CH₂-(CH₂)₃-NH-CH₂), 3.12 (m, 2H, NH-(CH₂)₂-CH₂-NH-CH₂), 3.09 (m, 2H, NH-(CH₂)₃-CH₂-NH-CH₂), 2.00 (tt, 2H, NH-CH₂-CH₂-CH₂-NH), 1.76 (m, 2H, NH-(CH₂)₂-CH₂-CH₂-NH-CH₂), 1.67 (m, 2H, NH-CH₂-CH₂-(CH₂)₂-NH-CH₂) ppm. ¹³C NMR (150 MHz, 298 K, D₂O): δ 156.9 (C=NH), 47.21 (NH-(CH₂)₃-CH₂-NH-CH₂), 44.83 (NH-(CH₂)₂-CH₂-NH-CH₂), 40.41 (NH-CH₂-(CH₂)₃-NH-CH₂), 38.19 (NH-CH₂-(CH₂)₂-NH-CH₂), 25.03 (NH-CH₂-CH₂-(CH₂)₂-NH-CH₂), 24.95 (NH-CH₂-CH₂-CH₂-NH), 22.83 (NH-(CH₂)₂-CH₂-CH₂-NH-CH₂) ppm.

### 4. Synthesis of First-generation Monomer 4 (^{Gu}M^{Gen.I}-4)

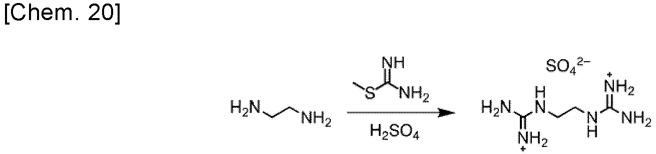

In a 200-milliliter three-necked round bottom flask that had been dried in an oven, S-methylisothiourea·0.5 H₂SO₄ (13.9 g, 0.1 mol) was dissolved in a mixed solution of water and ethanol (v/v 1:3). 16 Milliliters of ethylenediamine was added to the solution. The reaction mixture was stirred for 24 hours to produce a white precipitate. The crude residue was washed with ethanol, and was recrystallized with a mixed solution of water and ethanol (v/v 1:1) to provide ^{Gu}M^{Gen.I}-4 that was a transparent crystal (yield: 96%, based on ethylenediamine). The product was identified by ¹H NMR and ¹³C NMR.
¹H NMR (600 MHz, 298 K, D₂O): δ 3.44 (s, 4H, CH₂-CH₂). ¹³C NMR (150 MHz, 298 K, D₂O): δ 159.95 (C=NH), 42.95 (CH₂-CH₂).

### 5. Synthesis of First-generation Monomer 5 (^{Gu}M^{Gen.I}-5)

^{Gu}M^{Gen.I}-5 was synthesized through the following two steps.

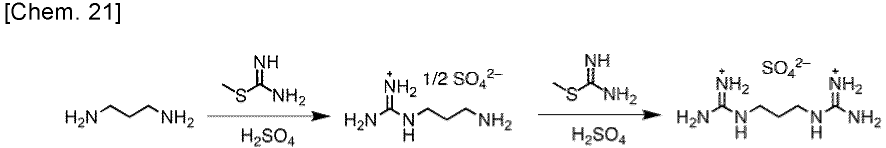

Step I: In a 200-milliliter three-necked round bottom flask that had been dried in an oven, S-methylisothiourea·0.5 H₂SO₄ (13.9 g, 0.1 mol) was dissolved in 50 mL of a mixed solution of water and ethanol (v/v 1:3). 10 Milliliters of 1,3-diaminopropane was added to the solution. After the mixture had been stirred for several minutes, a mixed solution of water and ethanol (v/v 1:3) was poured into the mixture, and stirring was further continued for 30 minutes. The crude residue was filtered, washed with water, and recrystallized to provide a desired crystalline product (yield: 72%, based on 1,3-diaminopropane). The product was identified by ¹H NMR.

¹H NMR (600 MHz, 298 K, D₂O): δ 3.31 (t, 4H, CH₂-NH), 3.08 (p, 2H, CH₂-NH₂), 1.98 (p, 2H, CH₂-CH₂-CH₂) ppm.

Step II: The product obtained in Step I (7.4 g) and S-methylisothiourea·0.5 H₂SO₄ (4.81 g, 34.5 mmol) were dissolved in 50 mL of water. 1.5 Milliliters of an aqueous solution (5 M) of sodium hydroxide was added dropwise to the mixture, and the whole was refluxed for 8 hours. The reaction mixture was cooled to room temperature. The flask was placed in a freezer at 4°C for 12 hours to produce a white precipitate. The crude product was filtered, washed with water, and recrystallized to provide ^{Gu}M^{Gen.I}-5 (yield: 38%, based on 1,3-diaminopropane). The product was identified by ¹H NMR and ¹³C NMR.

¹H NMR (600 MHz, 298 K, D₂O): δ 3.28 (t, 4H, CH₂-NH), 1.89 (p, 2H, CH₂-CH₂-CH₂) ppm.

¹³C NMR (150 MHz, 298 K, D₂O): δ 156.85 (C=NH), 38.34 (CH₂-NH), 26.97 (CH₂-CH₂-CH₂) ppm.

### 6. Synthesis of First-generation Monomers 6, 7, and 8 (^{Gu}M^{Gen.I}-6, ^{Gu}M^{Gen.I}-7, and ^{Gu}M^{Gen.I}-8)

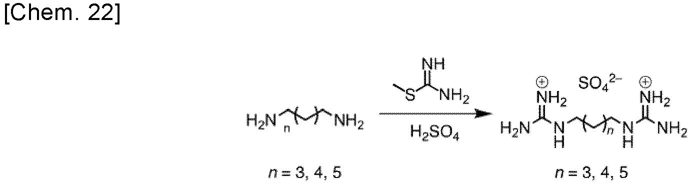

The series of guanidium monomers (from ^{Gu}M^{Gen.I}-6 to ^{Gu}M^{Gen.I}-8) was synthesized in accordance with the same synthesis protocol as that of ^{Gu}M^{Gen.I}-5. In a flask, an aliphatic diamine (0.5 mol) was added to a solution of S-methylisothiourea·0.5 H₂SO₄ (250.5 g, 1.8 mol), and the reaction mixture was stirred at 105°C for 3 days. The reaction mixture was cooled to room temperature. The flask was placed in a freezer at 4°C for 12 hours to produce a white precipitate. The crude product was filtered, washed with water, and recrystallized to provide each of desired products (from ^{Gu}M^{Gen.I}-6 to ^{Gu}M^{Gen.I}-8). The products were each identified by ¹H NMR and ¹³C NMR. ^{Gu}M^{Gen.I}-6 (n=3)

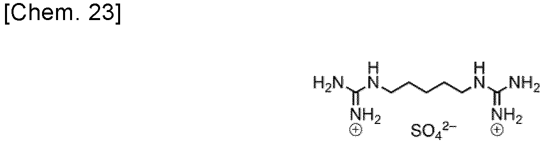

¹H NMR (600 MHz, 298 K, D₂O): δ 3.22 (t, 4H, CH₂-NH), 1.56 (t, 4H, CH₂-CH₂-CH₂-CH₂) ppm.
^{Gu}M^{Gen.I}-7 (n=4)

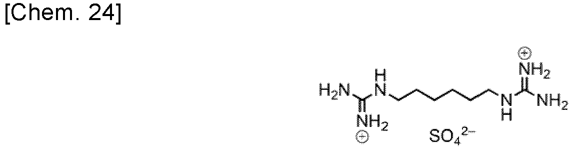

¹H NMR (600 MHz, 298 K, D₂O): δ 3.19 (t, 4H, CH₂-NH), 1.62 (t, 4H, CH₂-CH₂-CH₂-CH₂-CH₂), 1.41 (p, 2H, CH₂-CH₂-CH₂-CH₂-CH₂) ppm. ¹³C NMR (150 MHz, 298 K, D₂O): δ 156.76 (C=NH), 40.89 (CH₂-NH), 26.97 (CH₂-CH₂-CH₂-CH₂-CH₂), 22.90 (CH₂-CH₂-CH₂-CH₂-CH₂) ppm. ^{Gu}M^{Gen.I}-8 (n=5)

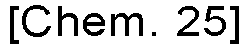

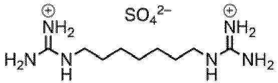

¹H NMR (600 MHz, 298 K, D₂O): δ 2.60 (t, 4H, CH₂-NH), 1.44 (t, H, NH-CH₂-CH₂), 1.33 (p, 6H, NH-CH₂-CH₂-CH₂-CH₂-CH₂) ppm. ¹³C NMR (150 MHz, 298 K, D₂O): δ 156.73 (C=NH), 41.20 (CH₂-NH), 27.82 (NH-CH₂-CH₂), 27.73 (NH-CH₂-CH₂-CH₂), 25.63 (NH-CH₂-CH₂-CH₂-CH₂) ppm.

### 7. Synthesis of First-generation Monomer 9 (^{Gu}M^{Gen.I}-9)

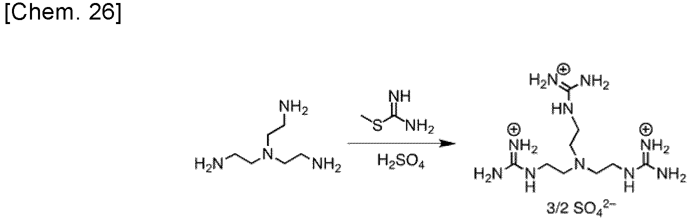

In a 200-milliliter three-necked round bottom flask that had been dried in an oven, S-methylisothiourea·0.5 H₂SO₄ (13.9 g, 0.1 mol) was dissolved in Milli-Q water. 5 Milliliters of bis(2-aminoethyl)ethane-1,2-diamine was added to the solution. The reaction mixture was refluxed for 24 hours to produce a white precipitate. The crude residue was washed with ethanol, and was recrystallized with Milli-Q water to provide ^{Gu}M^{Gen.I}-9 that was a transparent crystal (yield: 96%, bis(2-aminoethyl)ethane-1,2-diamine). The product was identified by ¹H NMR and ¹³C NMR.
¹H NMR (600 MHz, 298 K, D₂O): δ 3.29 (t, 4H, CH₂-NH), 2.70 (t, 4H, N-CH₂-CH₂) ppm. ¹³C NMR (150 MHz, 298 K, D₂O): δ 156.93 (C=NH), 52.33 (CH₂-NH), 38.92 (N-CH₂-CH₂) ppm.

### 8. Synthesis of First-generation Monomer 10 (^{Gu}M^{Gen.I}-10)

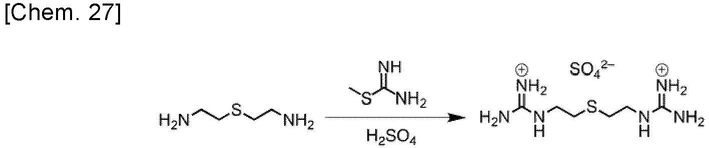

In a 300-milliliter three-necked round bottom flask that had been dried in an oven, S-methylisothiourea·0.5 H₂SO₄ (13.9 g, 0.1 mol) was dissolved in 80 mL of Milli-Q water. 30.6 Milliliters of 2,2'-thiobis(ethan-1-amine) (0.25 mol) was added to the solution. The mixture was refluxed for 3 days. The reaction mixture was cooled to room temperature to produce a white precipitate. The precipitate was sucked and recrystallized from water to provide ^{Gu}M^{Gen.I}-10 that was a white crystal (yield: 72%, based on 2,2'-thiobis(ethan-1-amine)). The product was identified by ¹H NMR and ¹³C NMR.
¹H NMR (600 MHz, 298 K, D₂O): δ 3.43 (t, 4H; NH-CH₂), 2.83 (t, 4H; CH₂-S) ppm. ¹³C NMR (150 MHz, 298 K, D₂O): δ 156.90 (C=N), 40.50 (NH-CH₂), 30.27 (CH₂-S) ppm.

### 9. Synthesis of First-generation Monomer 11 (^{Gu}M^{Gen.I}-11)

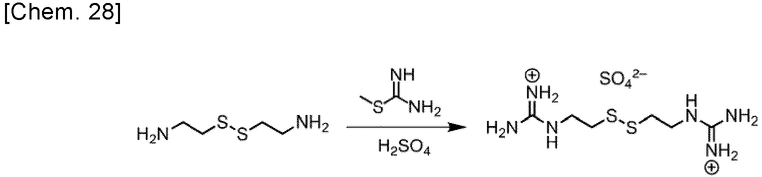

In a 300-milliliter three-necked round bottom flask that had been dried in an oven, S-methylisothiourea·0.5 H₂SO₄ (13.9 g, 0.1 mol) was dissolved in 80 mL of Milli-Q water. 40 Milliliters of 2,2'-disulfanediyldiethanamine (0.25 mol) was added to the solution. The mixture was refluxed for 24 hours. The reaction mixture was cooled to room temperature to produce a white precipitate. The precipitate was sucked and recrystallized from water to provide ^{Gu}M^{Gen.I}-11 that was a white crystal (yield: 96%, based on 2,2'-disulfanediyldiethanamine). The product was identified by ¹H NMR and ¹³C NMR.
¹H NMR (600 MHz, 298 K, D₂O): δ 3.60 (t, 4H; NH-CH₂), 3.38 (t, 4H; CH₂-S) ppm. ¹³C NMR (150 MHz, 298 K, D₂O): δ 156.90 (C=N), 39.77 (NH-CH₂), 36.11 (NH-CH₂-CH₂) ppm.

### 10. Synthesis of First-generation Monomer 12 (^{Gu}M^{Gen.I}-12)

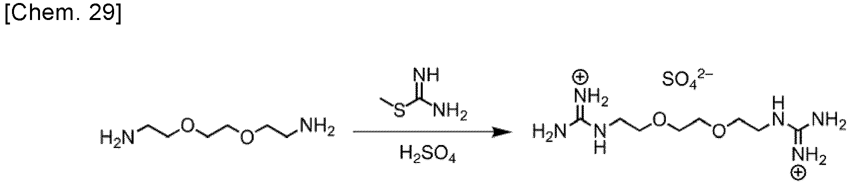

In a 300-milliliter three-necked round bottom flask that had been dried in an oven, 2,2'-(ethane-1,2-diylbis(oxy))bis(ethan-1-amine) (38.5 mL, 0.25 mol) was added to a solution obtained by dissolving S-methylisothiourea·1/2 H₂SO₄ (13.9 g, 0.1 mol) in Milli-Q water. The mixture was refluxed for 24 hours. The refluxed product was concentrated with a small evaporator, and was then left at rest at 4°C for 3 days. A transparent precipitate was produced, and was washed with cold ethanol. The recrystallization of the washed product from a mixture of water and ethanol produced ^{Gu}M^{Gen.I}-12 that was a transparent crystal (yield: 32%, based on 2,2'-(ethane-1,2-diylbis(oxy))bis(ethan-1-amine)). The product was identified by ¹H NMR and ¹³C NMR. ¹H NMR (600 MHz, 298 K, D₂O): δ 3.72 (t, 4H; NH-CH₂), 3.70 (p, 4H; NH-CH₂-CH₂-O), 3.41 (t, 4H; O-CH₂-CH₂-O) ppm.
¹³C NMR (150 MHz, 298 K, D₂O): δ 157.30 (C=N), 69.68 (CH₂-O), 68.81 (O-CH₂-CH₂-O), 41.16 (NH-CH₂) ppm.

### 11. Synthesis of Second-generation Monomer 1 (^{Gu}M^{Gen.II}-1)

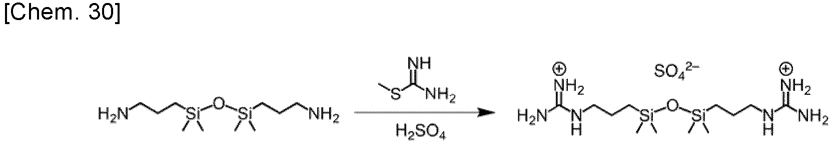

In a 1,000-milliliter three-necked round bottom flask that had been dried in an oven, S-methylisothiourea·0.5 H₂SO₄ (40 g, 0.3 mol) was dissolved in 700 mL of Milli-Q water. 14 Milliliters of 1,3-bis(3-aminopropyl)tetraethylsiloxane was added to the solution. The reaction mixture was refluxed for 24 hours. The reaction mixture was cooled to room temperature, and the crude residue was filtered and washed with cold water to provide ^{Gu}M^{Gen.II}-1 (yield: 53%, based on 1,3-bis(3-aminopropyl)tetraethylsiloxane). The product was identified by ¹H NMR and ¹³C NMR. ¹H NMR (600 MHz, 298 K, D₂O): δ 3.18 (t, 4H; NH-CH₂), 1.62 (p, 4H; NH-CH₂-CH₂), 0.61 (t, 4H; CH₂-O-Si), 0.14 (s, 12H; Si-CH₃) ppm. ¹³C NMR (150 MHz, 298 K, D₂O): δ 156.67 (C=N), 43.70 (NH-CH₂), 22.14 (NH-CH₂-CH₂), 14.10 (CH₂-O-Si), 0.70 (Si-CH₃) ppm.

### 12. Synthesis of Second-generation Monomer 2 (^{Gu}M^{Gen.II}-2)

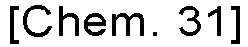

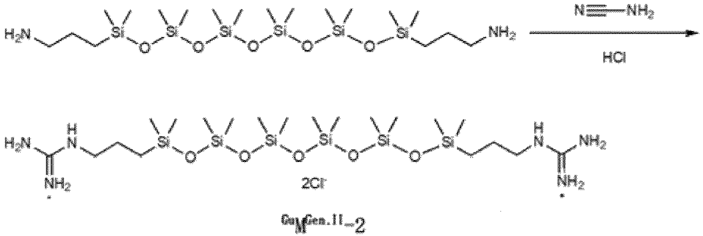

In a 100-milliliter three-necked round bottom flask that had been dried in an oven, 3,3'-(1,1,3,3,5,5,7,7,9,9,11,11-dodecamethylhexasiloxane-1,11-diyl)bis(propan-1-amine) (5.4 mL, 10 mmol) was dissolved in a solution of hydrochloric acid (1.5 mL, wt.%=36.5%) in ethanol (20 mL). Cyanamide (1.6 g, 0.04 mol) was added to the solution. The reaction mixture was refluxed for 4 hours. The reaction mixture was cooled to room temperature, and was then evaporated under reduced pressure until the mixture was dried. Thus, a viscous liquid was obtained. The resultant viscous liquid was rinsed with warm water (70°C, 100 mL×3) to provide ^{Gu}M^{Gen.II}-2 that was a liquid (yield: 12%, based on 3,3'-(1,1,3,3,5,5,7,7,9,9,11,11-dodecamethylhexasiloxane-1,11-diyl)bis(propan-1-amine)). The product was identified by ¹H NMR.
¹H NMR (600 MHz, 298 K, MeOD): δ 2.92 (t, 4H; NH-CH₂), 1.71 (p, 4H; NH-CH₂-CH₂), 0.64 (t, 4H; CH₂-O-Si), 0.12 (m, 36H; Si-CH₃) ppm.

### Example 2 Synthesis of Supramolecular Polymer Glass (SPG)

### 1. Synthesis of First-generation Supramolecular Polymer Glass (^{Gen.I}SPG)

A first-generation supramolecular polymer glass is synthesized by using each of the first-generation monomers produced in Example 1. The synthesis of the first-generation supramolecular polymer glass can be performed in an aqueous system, and neither any organic solvent nor any expensive rare earth metal (rare metal) catalyst is involved therein. In each of Examples 2 and 4 to 9 below, unless otherwise stated, a supramolecular polymer glass was synthesized in a flask as in Example 1.

An aqueous solution of each of the guanidinium-based first-generation monomers (^{Gu}M^{Gen.I}) produced in Example 1 was added to an aqueous solution of sodium hexametaphosphate (or sodium trimetaphosphate) so that the theoretical mixing molar ratio of a guanidium monomer to a phosphodiester became 1:1. Irrespective of which one of the first-generation monomers was used, the mixed aqueous solution immediately underwent a liquid-liquid phase separation to produce a cloudy supramolecular polymer emulsion (FIG. 2A and FIG. 2B, in FIG. 2A, an aqueous layer 10 and a layer of a viscous liquid 11 formed of a supramolecular polymer glass undergo a phase separation at an interface 12). The supramolecular polymer emulsion was centrifuged and concentrated to provide a viscous liquid. The liquid was rinsed with pure water and vacuum-dried to provide a first-generation supramolecular polymer glass (^{Gen.I}SPG) in 98% yield.

### 2. Synthesis of Second-generation Supramolecular Polymer Glass (^{Gen.II}SPG)

A second-generation supramolecular polymer glass is synthesized by using each of the second-generation monomers.

An aqueous solution of each of the guanidinium-based second-generation monomers (^{Gu}M^{Gen.II}) produced in Example 1 was added to an aqueous solution of sodium hexametaphosphate (or sodium trimetaphosphate) so that the theoretical mixing molar ratio of a guanidium monomer to a phosphodiester became 1:1. Irrespective of which one of the second-generation monomers was used, the mixed aqueous solution immediately underwent a liquid-liquid phase separation to produce a cloudy supramolecular polymer emulsion. The supramolecular polymer emulsion was centrifuged and concentrated to provide a viscous liquid. The liquid was rinsed with pure water and vacuum-dried to provide a second-generation supramolecular polymer glass (^{Gen.II}SPG) in 72% yield.

### 3. Synthesis of Third-generation Supramolecular Polymer Glass (^{Gen.III}SPG)

A third-generation supramolecular polymer glass is synthesized by using both of a first-generation monomer and a second-generation monomer.

An aqueous solution obtained by blending one each of the guanidinium-based first-generation monomers (^{Gu}M^{Gen.I}) and second-generation monomers (^{Gu}M^{Gen.II}) produced in Example 1 in combination was added to an aqueous solution of sodium hexametaphosphate (or sodium trimetaphosphate) so that the theoretical mixing molar ratio of guanidium monomers to a phosphodiester became 1:1. The mixed aqueous solution immediately underwent a liquid-liquid phase separation to produce a cloudy supramolecular polymer emulsion. The supramolecular polymer emulsion was centrifuged and concentrated to provide a viscous liquid. The liquid was rinsed with pure water and vacuum-dried to provide a third-generation supramolecular polymer glass (^{Gen.III}SPG) in 90% yield.

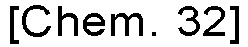

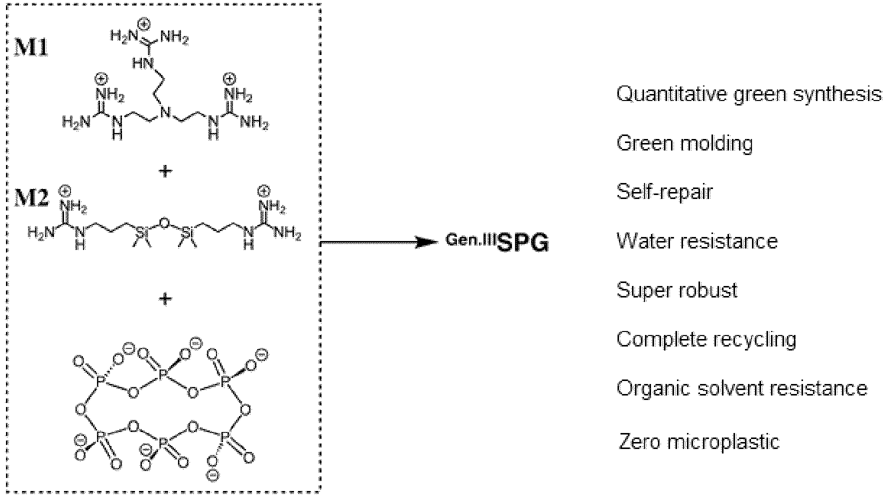

### Example 3 Evaluations of Physical Properties of Supramolecular Polymer Glass (SPG)

### 1. Light transmittance of Supramolecular Polymer Glass (SPG)

A SPG film having a size measuring 100 mm long by 100 mm wide by 0.5 mm thick was produced, and the light transmittance of the SPG film was measured by the transmission mode of a UV-visible spectrometer.

### (Result)

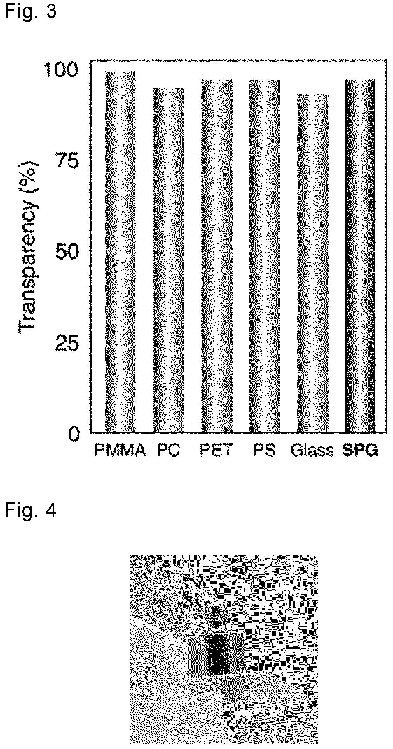

As a result, the optical transparency of the SPG film fell within the range of from 90% to 97%, though the optical transparency varied depending the molecular structures of its monomers. Accordingly, the light transmittance thereof was comparable to those of a commercial transparent resin film (polymethyl methacrylate (PMMA)), polycarbonate (PC), polyethylene terephthalate (PET), polystyrene (PS), and an inorganic glass (Glass) (FIG. 3).

### 2. Mechanical Characteristics of Supramolecular Polymer Glass (SPG)

A SPG film having a size measuring 100 mm long by 100 mm wide by 0.5 mm thick was produced. The film was mounted on a metal plate, and its Young's modulus was determined with an indentation hardness tester ENT-NEXUS (ELIONIX Inc.). A diamond indenter tip was used in the indentation test. The maximum load was set to 50 mN, and a loading/unloading rate was set to 2.5 mN/s. The duration of the maximum load was set to 5 s. The Young's modulus and indentation hardness of the film were determined with the tester from a curve at the time of unloading. A measurement temperature was 25°C.

The tensile strength of a commercial material is a value described in each of Wikipedia and a known literature. A sample measuring 35 mm long by 2 mm wide by 0.5 mm thick was produced, and its tensile strength was measured with a tensile machine at a test speed of 10 mm/s.

### (Result)

As shown in FIG. 4, even when a weight is mounted on the first-generation supramolecular polymer glass, the glass can maintain its shape and withstand the load.

As shown in FIG. 5, unexpectedly, the Young's modulus of the second-generation supramolecular polymer glass was more than 5 GPa, and the Young's modulus of the first-generation supramolecular polymer glass was more than 15 GPa. Accordingly, the Young's moduli of the SPG films were higher than those of commercial resin films.

As shown in FIG. 6, the tensile strengths of the first-generation and second-generation supramolecular polymer glasses fell within the range of from 20 GPa to 50 GPa, and were hence not inferior to those of the commercial resin films.

### 3. Mechanical Characteristics of Third-generation Supramolecular Polymer Glass (SPG)

The Young's modulus of the third-generation SPG was also measured under the same conditions as those in the above-mentioned section "2. Mechanical Characteristics of Supramolecular Polymer Glass (SPG)." However, a measurement temperature was 30°C.

### (Result)

As shown in FIG. 7, although the Young's modulus of the second-generation SPG (^{Gen.2}SPG, the leftmost bar) was low, and the Young's modulus of the first-generation SPG (^{Gen.1}SPG, the rightmost bar) was high, the mixing of the first-generation SPG and the second-generation SPG was able to adjust a Young's modulus.

### 4. Processability of Supramolecular Polymer Glass (SPG)

The glass transition temperatures (Tg) of the first-generation to third-generation SPGs produced in Example 2 ranged from 35°C to 125°C. As in a related-art synthetic resin such as PET, each of the SPGs was able to be processed into various shapes or patterns by hot pressing at temperatures around its Tg.

### 5. Self-repairability of Supramolecular Polymer Glass (SPG)

After the ^{Gu}M^{Gen.I}-2-based SPG produced in Example 2 had been broken into two pieces by applying a pressure thereto under ambient conditions (20°C and a humidity of 60%), the SPG was able to completely self-repair. After the self-repair, the mechanical properties of the SPG were not impaired. In addition, each of the SPGs produced in Example 2 can self-repair with the aid of water or humidity. Specifically, the first-generation supramolecular polymer glass ^{Gen.I}SPG was able to self-repair by being pressed under a high-humidity (RH80%) condition for 30 minutes, and the second-generation supramolecular polymer glass ^{Gen.II}SPG was able to self-repair by being pressed under water spray for 20 minutes.

### 6. Underwater Processability of First-generation Supramolecular Polymer Glass (^{Gen.I}SPG)

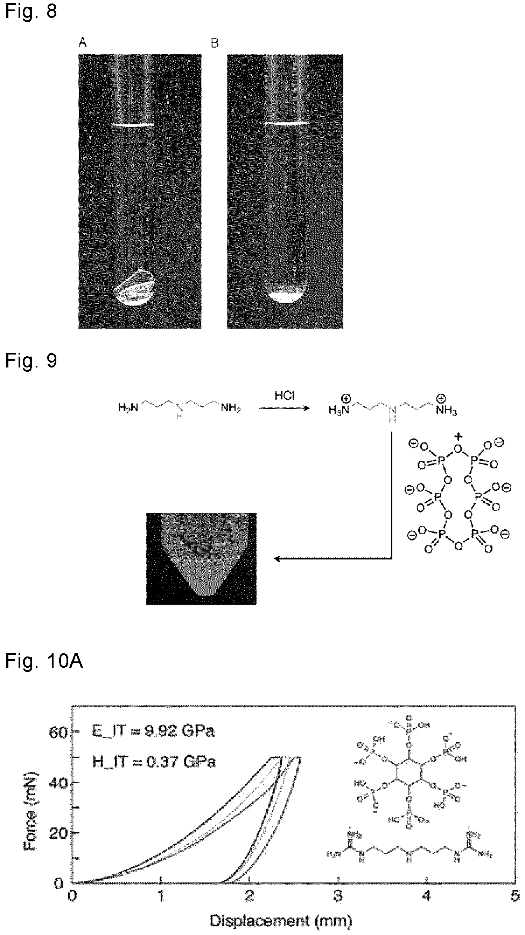

When each first-generation supramolecular polymer glass ^{Gen.I}SPG produced in Example 2 was immersed in water, the glass gradually softened (FIG. 8A), and became a viscous supramolecular polymer liquid in several hours (FIG. 8B). When the viscous liquid was vacuum-dried in a Teflon (trademark) container at 80°C for 6 hours, a ^{Gen.I}SPG having a mechanical strength was obtained with neither a monomer loss nor a mechanical property loss. The ^{Gen.I}SPG cohesively softens even by being sprayed with water, and hence can be molded into various structural products.

### Example 4 Synthesis of Supramolecular Polymer Glass through Use of Ammonium-based Monomer

An ammonium group of an ammonium-based molecule interacts with an oxyanion, such as a carboxylate group or a phosphodiester group, to form a crosslinked supramolecular network, thereby producing a SPG.

As a typical example, a commercial low-molecular weight material having a di/tri/tetraamino group can form a supramolecular polymer with an oxyanion through a salt bridge.

As shown in FIG. 9, a diamine monomer was dissolved at 0.06 M in ethanol, and a 0.2 M sodium hexametaphosphate solution was added to the prepared diamine monomer solution. The solution immediately became cloudy, and a liquid-liquid phase separation phenomenon was instantly observed. As a result of centrifugal separation at 12,000 r/min for 10 minutes, such a clear liquid-liquid phase separation as represented by a dotted line in a photograph in the lower left of FIG. 9 was observed. The lower viscous liquid was washed with 50 mL of deionized water three times. After the washed product had been vacuum-dried at 80°C for 3 hours, a transparent glass was obtained in about 98% yield.

### Example 5 Synthesis of SPG through Use of Recyclable Raw Material Monomer

A water-soluble low-molecular weight biomolecule or biopolymer is stored in plenty on the earth, and the use of our strategy may enable such molecule to form a SPG. In this Example, a SPG was produced by using the following two recyclable raw materials as raw material monomers for an oxoanion: phytic acid and alginic acid.

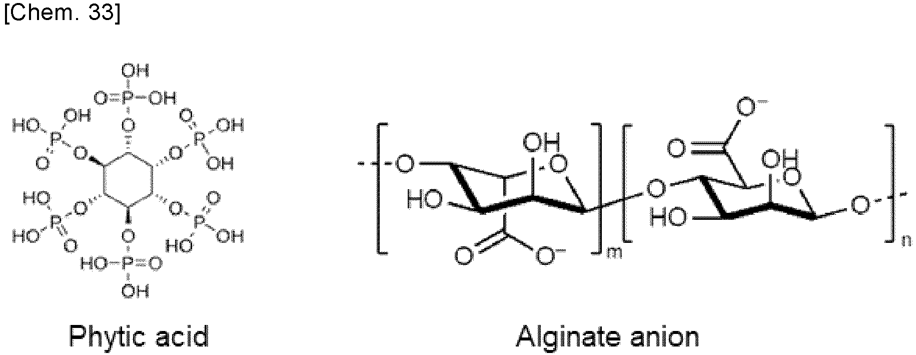

Phytic acid is a dihydrogen phosphate of the hexaploid of inositol, and is the main storage form of phosphorus in grains, beans, oilseeds, and nuts. Alginic acid is a naturally occurring edible polysaccharide purified from brown algae present in the natural world and specific bacteria. Alginic acid is rich in carboxyl groups, and hence can form salt bridges with an ammonium group and a guanidium group. In addition, alginic acid can be incorporated as an additive for reinforcing the mechanical characteristics of a SPG into the ^{Gen.1}SPG or the ^{Gen.2}SPG.

### 1-1. Phytic Acid-based SPG

An aqueous solution of a guanidinium-based monomer was added to an aqueous solution of phytic acid so that a stoichiometric molar ratio between a guanidino group and a phosphate group (a guanidinium ion and a phosphate ion) became 1:1. The mixed aqueous solution instantly underwent a liquid-liquid phase separation to provide a cloudy supramolecular polymer emulsion. The supramolecular polymer emulsion was subjected to centrifugal separation to be concentrated into a viscous liquid. When the viscous liquid was washed with Milli-Q water, and was then vacuum-dried, a supramolecular polymer glass (SPG) was obtained. The SPG was able to be processed into various shapes or patterns by hot pressing.

### 1-2. Characteristics of Phytic Acid-based SPG

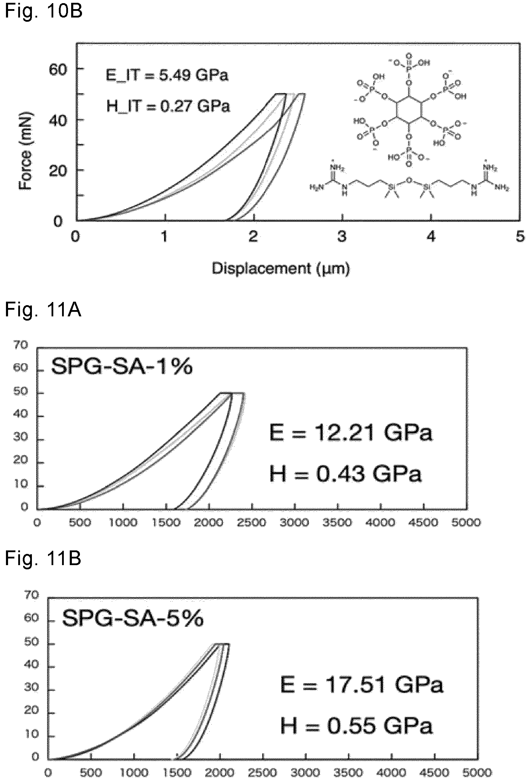

The mechanical characteristics of the SPG film produced in the section 1-1 were determined by an indenter test with ENT-NEXUS (ELIONIX Inc.) under the same measurement conditions as those of the section 2. of Example 3. A diamond indenter tip having a Berkovich shape was used in the indentation experiment. In the case of the combination of phytic acid and ^{Gu}M^{Gen.II}-1, the Young's modulus of the SPG was 5.5 GPa (FIG. 10A). In addition, when a SPG was produced by using ^{Gu}M^{Gen.I}-2 instead of ^{Gu}M^{Gen.II}-1, the Young's modulus of the SPG increased to 10 GPa (FIG. 10B).

### 2-1. Alginic Acid-based SPG

An amine-based or guanidine-based monomer was added to a dilute aqueous solution of alginic acid so that a stoichiometric molar ratio between a guanidino group/amino group and a carboxyl group (a guanidinium ion/ammonium ion and a carboxylate ion) became 1:1. The mixed aqueous solution instantly underwent a liquid-liquid phase separation to provide a cloudy supramolecular polymer emulsion. The supramolecular polymer emulsion was subjected to centrifugal separation to be concentrated into a viscous liquid. When the viscous liquid was washed with Milli-Q water, and was then dried under a vacuum, a supramolecular polymer glass was obtained.

### 2-2. Alginic Acid-reinforced SPG

An amine-based or guanidine-based monomer was added to a mixed aqueous solution of alginic acid and hexametaphosphoric acid so that a stoichiometric molar ratio between a guanidino group/amino group and a carboxyl group (a guanidinium ion/ammonium ion and a carboxylate ion) became 1:1. The mixed aqueous solution instantly underwent a liquid-liquid phase separation to provide a cloudy supramolecular polymer emulsion. The supramolecular polymer emulsion was subjected to centrifugal separation to be concentrated into a viscous liquid. When the viscous liquid was washed with Milli-Q water, and was then dried under a vacuum, a supramolecular polymer glass was obtained.

### 2-3. Result

The mechanical characteristics of the SPG film were determined by an indenter test with ENT-NEXUS (ELIONIX Inc.) under the same measurement conditions as those of the section 2. of Example 3. A diamond indenter tip having a Berkovich shape was used in the indentation experiment. The mechanical characteristics of the alginic acid-based SPG and the alginic acid-reinforced SPG are shown in FIG. 11A to FIG. 11C.

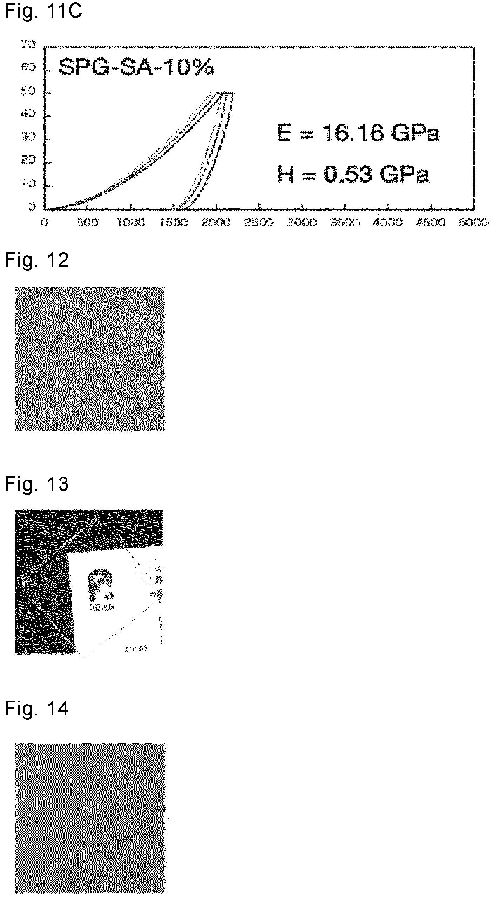

In each sample, the Young's modulus of the SPG was as high as more than 10 GPa (12.21 GPa, 17.51 GPa, and 16.16 GPa in FIG. 11A, FIG. 11B, and FIG. 11C, respectively).

### Example 6 Synthesis of SPG through use of Natural Polysaccharide

An aqueous solution of chondroitin sulfate and an aqueous solution of each of the guanidinium-based first-generation monomers (^{Gu}M^{Gen.I}) produced in Example 1 were mixed so that the theoretical mixing molar ratio of a guanidium monomer to an anionic functional group in chondroitin sulfate became 1:1. The mixed aqueous solution underwent a liquid-liquid phase separation to produce a cloudy supramolecular polymer emulsion (FIG. 12). The supramolecular polymer emulsion was centrifuged and concentrated to provide a viscous liquid. The liquid was rinsed with pure water and vacuum-dried to provide a supramolecular polymer glass in 95% yield (FIG. 13).

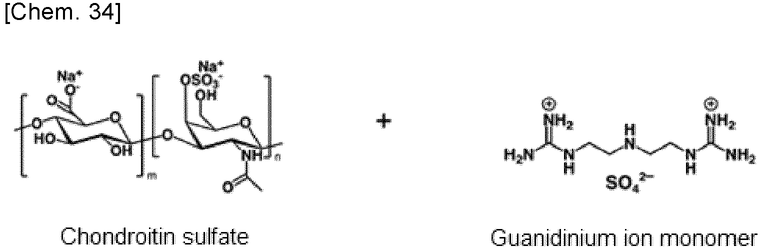

### Example 7 Synthesis of SPG through use of Natural Polysaccharide

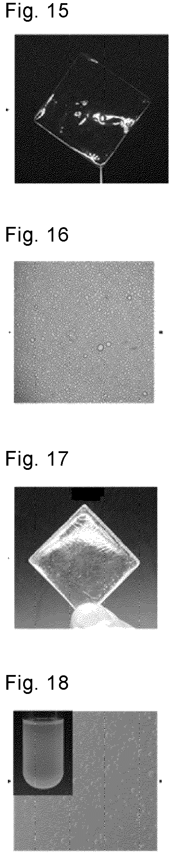

An aqueous solution of sodium heparin sulfate and an aqueous solution of each of the guanidinium-based first-generation monomers (^{Gu}M^{Gen.I}) produced in Example 1 were mixed so that the theoretical mixing molar ratio of a guanidium monomer to an anionic functional group in sodium heparin sulfate became 1:1. The mixed aqueous solution underwent a liquid-liquid phase separation to produce a cloudy supramolecular polymer emulsion (FIG. 14). The supramolecular polymer emulsion was centrifuged and concentrated to provide a viscous liquid. The liquid was rinsed with pure water and vacuum-dried to provide a supramolecular polymer glass in 98% yield (FIG. 15).

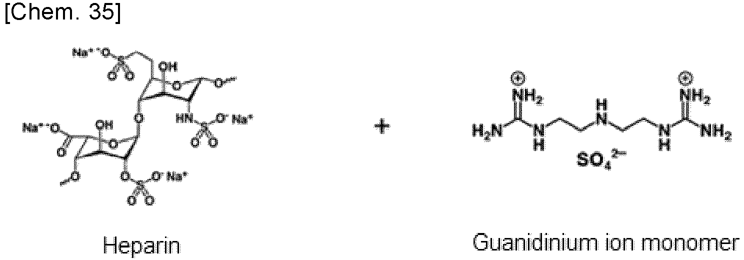

### Example 8 Synthesis of SPG through use of Synthetic Polysaccharide

An aqueous solution of sodium dextran sulfate and an aqueous solution of each of the guanidinium-based first-generation monomers (^{Gu}M^{Gen.I}) produced in Example 1 were mixed so that the theoretical mixing molar ratio of a guanidium monomer to an anionic functional group in sodium dextran sulfate became 1:1. The mixed aqueous solution underwent a liquid-liquid phase separation to produce a cloudy supramolecular polymer emulsion (FIG. 16). The supramolecular polymer emulsion was centrifuged and concentrated to provide a viscous liquid. The liquid was rinsed with pure water and vacuum-dried to provide a supramolecular polymer glass in 100% yield (FIG. 17).

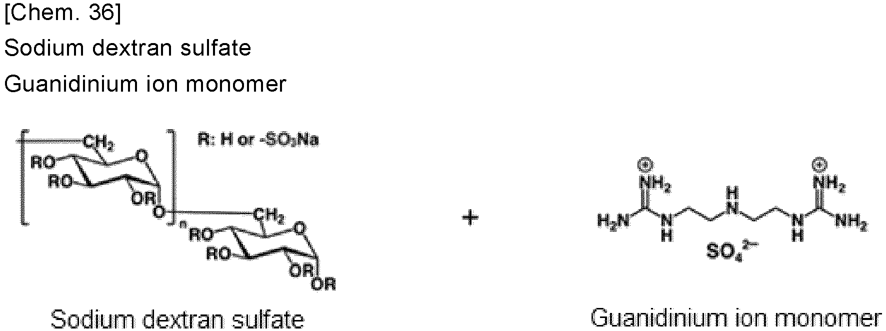

### Example 9 Synthesis of SPG through use of Synthetic Polysaccharide

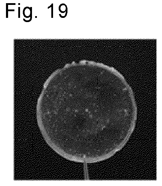

An aqueous solution of β-cyclodextrin (product number: CAS7585-39-9) in which part of the hydrogen atoms of hydroxy groups were each substituted with -SO₃Na and an aqueous solution of each of the guanidinium-based first-generation monomers (^{Gu}M^{Gen.I}) produced in Example 1 were mixed so that the theoretical mixing molar ratio of a guanidium monomer to an anionic functional group in β-cyclodextrin became 1:1. The mixed aqueous solution underwent a liquid-liquid phase separation to produce a cloudy supramolecular polymer emulsion (FIG. 18). The supramolecular polymer emulsion was centrifuged and concentrated to provide a viscous liquid. The liquid was rinsed with pure water and vacuum-dried to provide a supramolecular polymer glass in 98% yield (FIG. 19).

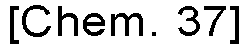

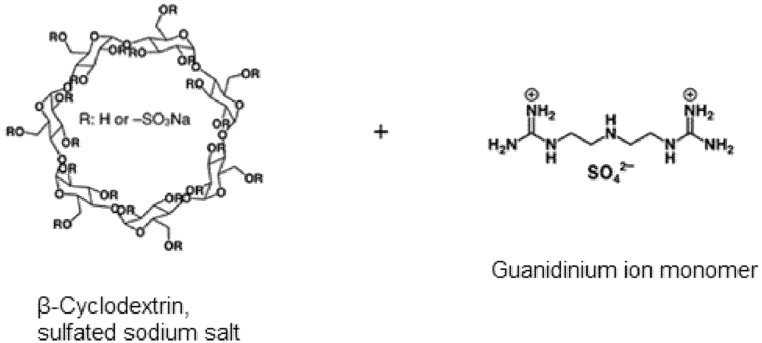

The synthesis of each of the supramolecular polymer glasses in Examples 2 and 4 to 9 was able to be performed under atmospheric pressure without need for the heating or cooling of the sample.

## Claims

1. A composite comprising:
an organic cation, which is obtained by ionization of a compound having at least two amino groups or guanidino groups; and
an oxyanion,
wherein the organic cation and the oxyanion are bonded to each other through an ionic bond and a hydrogen bond.

2. The composite according to claim 1, wherein the organic cation and the oxyanion are bonded to each other through an ionic bond and a hydrogen bond represented by one or two or more kinds out of the following formulae (1) to (4): 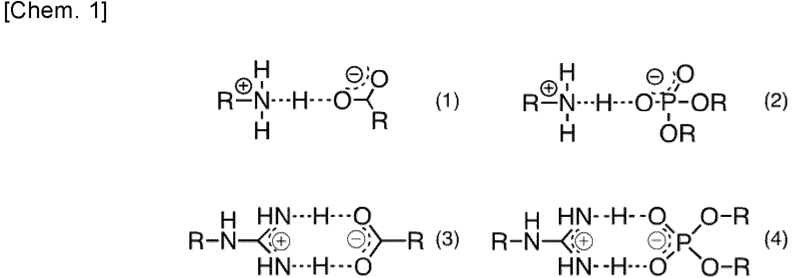 in each of the formulae (1) to (4), R represents an arbitrary monovalent organic group.

3. The composite according to claim 1, wherein the organic cation is an organic cation obtained by ionization of a guanidine compound represented by the following formula (6): 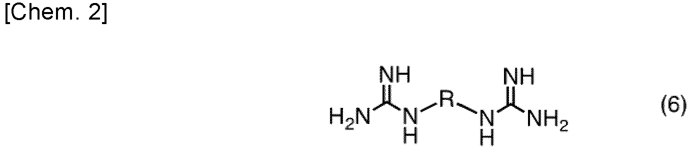 where R represents a substituted or unsubstituted hydrocarbon chain, and
when the hydrocarbon chain is substituted, part of methylene groups of the hydrocarbon chain are each substituted with a group selected from the group consisting of: -NH-; a -N(alkyl group)-; -O-; -COO-; -O-COO-; -NHCO-; -S-; a cycloalkane; a cycloalkanone; benzene; a group represented by the formula (7); and a substituted or unsubstituted -N(guanidylalkylene group)-, and when the - N(guanidylalkylene group)- is substituted, part of methylene groups of a guanidylalkylene group are each substituted with the same group as the group substituting each of part of the methylene groups of the hydrocarbon chain; 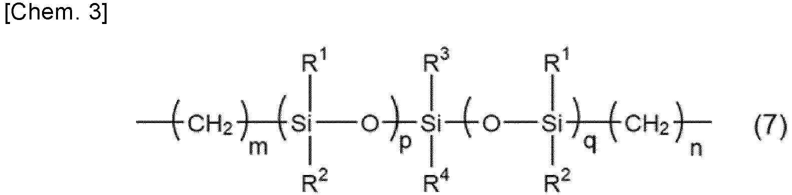 where R₁ and R₂ each independently represent an alkyl group having 1 to 6 carbon atoms or a phenyl group, R₃ and R₄ each independently represent an alkyl group having 1 to 6 carbon atoms or a phenyl group, "m" represents from 1 to 6, "n" represents from 1 to 6, "p" represents an integer in a range of from 0 to 20, and "q" represents an integer in a range of from 0 to 20, provided that p+q is an integer of 1 or more.

4. The composite according to claim 3, wherein the guanidine compound contains the following compound (I), the following compound (II), or both thereof:
(I) a compound represented by the formula (6) in which A represents a substituted or unsubstituted hydrocarbon chain, and when the hydrocarbon chain is substituted, part of methylene groups of the hydrocarbon chain are each substituted with a group selected from the group consisting of: -NH-; a -N(alkyl group)-; -O-; -COO-; -O-COO-; - NHCO-; -S-; a cycloalkane; a cycloalkanone; benzene; and a substituted or unsubstituted -N(guanidylalkylene group)-, and when the -N(guanidylalkylene group)- is substituted, part of methylene groups of a guanidylalkylene group are each substituted with the same group as the group substituting each of part of the methylene groups of the hydrocarbon chain, provided that a compound in which part of the methylene groups of the hydrocarbon chain are each substituted with a group represented by the formula (7) is excluded; and
(II) a compound represented by the formula (6) in which A represents a substituted hydrocarbon chain, and part of methylene groups of the hydrocarbon chain are each substituted with a group represented by the formula (7).

5. The composite according to claim 1, wherein the oxyanion is an oxyanion of sulfur, phosphorus, silicon, or carbon.

6. The composite according to claim 5, wherein the oxyanion is a polyoxyanion.

7. The composite according to claim 5, wherein the oxyanion is a cyclic phosphate anion represented by the following formula (10), a linear phosphate anion represented by the following formula (11), an anion of phytic acid, or an anion of a carboxylic acid: 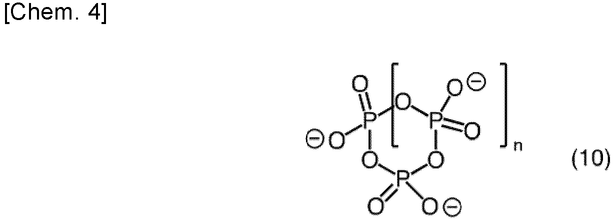 where "n" represents 1 or 4; 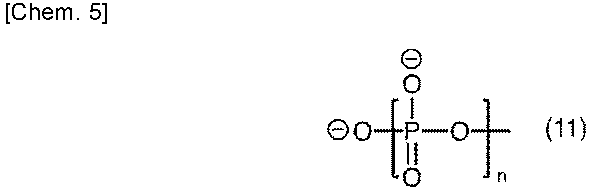 where "n" represents an integer of from 1 to 1,000.

8. The composite according to claim 1, wherein the oxyanion is an oxyanion produced by electrolytic dissociation of a polysaccharide having an anionic functional group.

9. The composite according to claim 1, wherein the composite is insoluble in an organic solvent.

10. The composite according to claim 1, wherein the composite is processable in water at 20°C.

11. The composite according to claim 1, wherein the composite has self-repairability.

12. The composite according to claim 1, wherein the composite having a thickness of 0.5 mm has a light transmittance of 90% or more at from 400 nm to 800 nm.

13. The composite according to claim 1, wherein the composite is a supramolecular plastic.

14. The composite according to claim 1, wherein the composite is a supramolecular polymer glass.

15. A composition comprising the composite of any one of claims 1 to 14.

16. An article comprising the composite of any one of claims 1 to 14.

17. A method of producing a composite, the method comprising mixing a compound having at least two amino groups or guanidino groups and an oxyanion-containing compound in water or an aqueous solution to produce a composite in which an organic cation formed by ionization of the compound having at least two amino groups or guanidino groups, and an oxyanion formed by ionization of the oxyanion-containing compound are bonded to each other through an ionic bond and a hydrogen bond.

18. A use of a compound having at least two amino groups or guanidino groups and an oxyanion-containing compound of sulfur, phosphorus, silicon, or carbon for production of a supramolecular polymer composite.
